# EUROPEAN PATENT APPLICATION

(11) **EP 2 112 231 A2**
(43) Date of publication of application: **28.10.2009**
(21) Application number: 09154158.1
(22) Date of filing: 21.05.1999
(51) Int. Cl.: C12Q 1/68, C07K 14/47

(54) **MarA family helix-turn-helix domains and methods of use**

(30) Priority: 22.05.1998 US 86497 P
(62) Divisional of application: 99925730.6
(71) Applicant: TRUSTEES OF TUFTS COLLEGE, Medford, MA 02155 (US)
(72) Inventor: Levy, Stuart B., Boston, MA 02116 (US)
(74) Representative: Nash, David Allan

(57) **Abstract**

An important advance in the battle against drug resistance by elucidating the domains of MarA which are critical in mediating its function. Accordingly, MarA family protein helix-turn-helix domains, mutant MarA family protein helix-turn-helix domains and methods of their use, for example, in screening assays to identify compounds which are useful as antiinfective agents and in screening assays to identify loci which are involved in mediating antibiotic resistance are described.

SEQUENCE LISTING

<110> Tufts University

<120> MarA Family Helix-Turn-Helix Domains and Their Methods
      of Use

<130> pkz-004cppc

<140>
<141>

<150> 60-086,497
<151> 1998-05-22

<160> 2

<170> PatentIn Ver. 2.0

<210> 1
<211> 7878
<212> DNA
<213> Escherichia coli

<400> 1
gttaactgtg gtggttgtca ccgcccatta cacggcatac agctatatcg agccttttgt 60
acaaaacatt gcgggattca gcgccaactt tgccacggca ttactgttat tactcggtgg 120
tgcgggcatt attggcagcg tgattttcgg taaactgggt aatcagtatg cgtctgcgtt 180
ggtgagtacg gcgattgcgc tgttgctggt gtgcctggca ttgctgttac ctgcggcgaa 240
cagtgaaata cacctcgggg tgctgagtat tttctggggg atcgcgatga tgatcatcgg 300
gcttggtatg caggttaaag tgctggcgct ggcaccagat gctaccgacg tcgcgatggc 360
gctattctcc ggcatattta atattggaat cggggcgggt gcgttggtag gtaatcaggt 420
gagtttgcac tggtcaatgt cgatgattgg ttatgtgggc gcggtgcctg cttttgccgc 480
gttaatttgg tcaatcatta tatttcgccg ctggccagtg acactcgaag aacagacgca 540
atagttgaaa ggcccattcg ggcctttttt aatggtacgt tttaatgatt tccaggatgc 600
cgttaataat aaactgcaca cccatacata ccagcaggaa tcccatcaga cgggagatcg 660
cttcaatgcc acccttgccc accagccgca taattgcgcc ggagctgcgt aggcttcccc 720
acaaaataac cgccaccagg aaaaagatca gcggcggcgc aaccatcagt acccaatcag 780
cgaaggttga actctgacgc actgtggacg ccgagctaat aatcatcgct atggttcccg 840
gaccggcagt acttggcatt gccagcggca caaaggcaat attggcactg ggttcatctt 900
ccagctcttc cgacttgctt ttcgcctccg gtgaatcaat cgctttctgt tgcggaaaga 960
gcatccgaaa accgataaac gcgacgatta agccgcctgc aattcgcaga ccgggaatcg 1020
aaatgccaaa tgtatccatc accagttgcc cggcgtaata cgccaccatc atgatggcaa 1080
atacgtacac cgaggccatc aacgactgac gattacgttc ggcactgttc atgttgcctg 1140
ccaggccaag aaataacgcg acagttgtta atgggttagc taacggcagc aacaccacca 1200
gccccaggcc aattgcttta aacaaatcta acattggtgg ttgttatcct gtgtatctgg 1260
gttatcagcg aaaagtataa ggggtaaaca aggataaagt gtcactcttt agctagcctt 1320
gcatcgcatt gaacaaaact tgaaccgatt tagcaaaacg tggcatcggt caattcattc 1380
atttgactta tacttgcctg ggcaatatta tcccctgcaa ctaattactt gccagggcaa 1440
ctaatgtgaa aagtaccagc gatctgttca atgaaattat tccattgggt cgcttaatcc 1500
atatggttaa tcagaagaaa gatcgcctgc ttaacgagta tctgtctccg ctggatatta 1560
ccgcggcaca gtttaaggtg ctctgctcta tccgctgcgc ggcgtgtatt actccggttg 1620
aactgaaaaa ggtattgtcg gtcgacctgg gagcactgac ccgtatgctg gatcgcctgg 1680
tctgtaaagg ctgggtggaa aggttgccga acccgaatga caagcgcggc gtactggtaa 1740
aacttaccac cggcggcgcg gcaatatgtg aacaatgcca tcaattagtt ggccaggacc 1800
tgcaccaaga attaacaaaa aacctgacgg cggacgaagt ggcaacactt gagtatttgc 1860
ttaagaaagt cctgccgtaa acaaaaaaga ggtatgacga tgtccagacg caatactgac 1920

gctattacca ttcatagcat tttggactgg atcgaggaca acctggaatc gccactgtca 1980
ctggagaaag tgtcagagcg ttcgggttac tccaaatggc acctgcaacg gatgtttaaa 2040
aaagaaaccg gtcattcatt aggccaatac atccgcagcc gtaagatgac ggaaatcgcg 2100
caaaagctga aggaaagtaa cgagccgata ctctatctgg cagaacgata tggcttcgag 2160
tcgcaacaaa ctctgacccg aaccttcaaa aattactttg atgttccgcc gcataaatac 2220
cggatgacca atatgcaggg cgaatcgcgc tttttacatc cattaaatca ttacaacagc 2280
tagttgaaaa cgtgacaacg tcactgaggc aatcatgaaa ccactttcat ccgcaatagc 2340
agctgcgctt attctctttt ccgcgcaggg cgttgcggaa caaaccacgc agccagttgt 2400
tacttcttgt gccaatgtcg tggttgttcc cccatcgcag gaacacccac cgtttgattt 2460
aaatcacatg ggtactggca gtgataagtc ggatgcgctc ggcgtgccct attataatca 2520
acacgctatg tagtttgttc tggccccgac atctcggggc ttattaactt cccaccttta 2580
ccgctttacg ccaccgcaag ccaaatacat tgatatacag cccggtcata atgagcaccg 2640
cacctaaaaa ttgcagaccc gttaagcgtt catccaacaa tagtgccgca cttgccagtc 2700
ctactacggg caccagtaac gataacggtg caacccgcca ggtttcatag cgtcccagta 2760
acgtccccca gatcccataa ccaacaattg tcgccacaaa cgccagatac atcagagaca 2820
agatggtggt catatcgata gtaaccagac tgtgaatcat ggttgcggaa ccatcgagaa 2880
tcagcgaggc aacaaagaag ggaatgattg ggattaaagc gctccagatt accagcgaca 2940
tcaccgccgg acgcgttgag tgcgacatga tctttttatt gaagatgttg ccacacgccc 3000
aactaaatgc tgccgccagg gtcaacataa agccgagcat cgccacatgc tgaccgttca 3060
gactatcttc gattaacacc agtacgccaa aaatcgctaa ggcgatcccc gccaattgtt 3120
tgccatgcag tcgctccccg aaagtaaacg cgccaagcat gatagtaaaa aacgcctgtg 3180
cctgtaacac cagcgaagcc agtccagcag gcataccgaa gttaatggca caaaaaagaa 3240
aagcaaactg cgcaaaactg atggttaatc cataccccag cagcaaattc agtggtactt 3300
tcggtcgtgc gacaaaaaag atagccggaa aagcgaccag cataaagcgc aaaccggcca 3360
gcatcagcgt ggcatgttat gaagccccac tttgatgacc acaaaattta gcccccatac 3420
gaccactacc agtagcgcca acaccccatc ttttcgcgac attctaccgc ctctgaattt 3480
catcttttgt aagcaatcaa cttagctgaa tttacttttc tttaacagtt gattcgttag 3540
tcgccggtta cgacggcatt aatgcgcaaa taagtcgcta tacttcggat ttttgccatg 3600
ctatttcttt acatctctaa aacaaaacat aacgaaacgc actgccggac agacaaatga 3660
acttatccct acgacgctct accagcgccc ttcttgcctc gtcgttgtta ttaaccatcg 3720
gacgcggcgc taccgtgcca tttatgacca tttacttgag tcgccagtac agcctgagtg 3780
tcgatctaat cggttatgcg atgacaattg cgctcactat tggcgtcgtt tttagcctcg 3840
gttttggtat cctggcggat aagttcgaca agaaacgcta tatgttactg gcaattaccg 3900
ccttcgccag cggttttatt gccattactt tagtgaataa cgtgacgctg gttgtgctct 3960
tttttgccct cattaactgc gcctattctg tttttgctac cgtgctgaaa gcctggtttg 4020
ccgacaatct ttcgtccacc agcaaaacga aaatcttctc aatcaactac accatgctaa 4080
acattggctg accatcggtc cgccgctcgg cacgctgttg gtaatgcaga gcatcaatct 4140
gcccttctgg ctggcagcta tctgttccgc gtttcccatg cttttcattc aaatttgggt 4200
aaagcgcagc gagaaaatca tcgccacgga aacaggcagt gtctggtcgc cgaaagtttt 4260
attacaagat aaagcactgt tgtggtttac ctgctctggt tttctggctt cttttgtaag 4320
cggcgcattt gcttcatgca tttcacaata tgtgatggtg attgctgatg gggattttgc 4380
cgaaaaggtg gtcgcggttg ttcttccggt gaatgctgcc atggtggtta cgttgcaata 4440
ttccgtgggc cgccgactta acccggctaa catccgcgcg ctgatgacag caggcaccct 4500
ctgtttcgtc atcggtctgg tcggttttat tttttccggc aacagcctgc tattgtgggg 4560
tatgtcagct gcggtattta ctgtcggtga aatcatttat gcgccgggcg agtatatgtt 4620
gattgaccat attgcgccgc cagaaatgaa agccagctat ttttccgccc agtctttagg 4680
ctggcttggt gccgcgatta acccattagt gagtggcgta gtgctaacca gcctgccgcc 4740
ttcctcgctg tttgtcatct tagcgttggt gatcattgct gcgtgggtgc tgatgttaaa 4800
agggattcga gcaagaccgt gggggcagcc cgcgctttgt tgatttaagt cgaacacaat 4860
aaagatttaa ttcagccttc gtttaggtta cctctgctaa tatctttctc attgagatga 4920
aaattaaggt aagcgaggaa acacaccaca ccataaacgg aggcaaataa tgctgggtaa 4980
tatgaatgtt tttatggccg tactgggaat aattttattt tctggttttc tggccgcgta 5040
tttcagccac aaatgggatg actaatgaac ggagataatc cctcacctaa ccggcccctt 5100
gttacagttg tgtacaaggg gcctgatttt tatgacggcg aaaaaaaacc gccagtaaac 5160
cggcggtgaa tgcttgcatg gatagatttg tgttttgctt ttacgctaac aggcattttc 5220
ctgcactgat aacgaatcgt tgacacagta gcatcagttt tctcaatgaa tgttaaacgg 5280
agcttaaact cggttaatca cattttgttc gtcaataaac atgcagcgat ttcttccggt 5340

ttgcttaccc tcatacattg cccggtccgc tcttccaatg accacatcca gaggctcttc 5400
aggaaatgcg cgactcacac ctgctgtcac ggtaatgttg atatgccctt cagaatgtgt 5460
gatggcatgg ttatcgacta actggcaaat tctgacacct gcacgacatg cttcttcatc 5520
attagccgct ttgacaataa tgataaattc ttcgcccccg tagcgataaa ccgtttcgta 5580
atcacgcgtc caactggcta agtaagttgc cagggtgcgt aatactacat cgccgattaa 5640
atgcccgtag tatcattaac caatttaaat cggtcaatat ccaacaacat taaataaaga 5700
ttcagaggct cagcgttgcg taactgatga tcaaaggatt catcaagaac ccgacgaccc 5760
ggcaatcccg tcaaaacatc catattgcta cggatcgtca gcaaataaat tttgtaatcg 5820
gttaatgccg cagtaaaaga aagcaacccc tcctgaaagg cgtcgaaatg cgcgtcctgc 5880
cagtgatttt caacaatagc cagcattaat tcccgaccac agttatgcat atgttgatgg 5940
gcagaatcca ttagccgaac gtaaggtaat tcatcgttat cgagtggccc cagatgatca 6000
atccaccgac caaactggca cagtccataa gaatggttat ccgttatttc tggcttactg 6060
gcatctctcg cgaccacgct gtgaaacata ctcaccagcc actggtagtg ggcatcgata 6120
gccttattga gatttaacaa gatggcatca atttccgttg tcttcttgat cattgccact 6180
cctttttcac agttccttgt gcgcgctatt ctaacgagag aaaagcaaaa ttacgtcaat 6240
attttcatag aaatccgaag ttatgagtca tctctgagat aacattgtga tttaaaacaa 6300
aatcagcgga taaaaaagtg tttaattctg taaattacct ctgcattatc gtaaataaaa 6360
ggatgacaaa tagcataacc caatacccta atggcccagt agttcaggcc atcaggctaa 6420
tttattttta tttctgcaaa tgagtgaccc gaacgacggc cggcgcgctt ttcttatcca 6480
gactgccact aatgttgatc atctggtccg gctgaacttc tcgtccatca aagacggccg 6540
caggaataac gacattaatt tcaccgctct tatcgcgaaa aacgtaacgg tcctctcctt 6600
tgtgagaaat caaattaccg cgtagtgaaa ccgaagcgcc atcgtgcatg gtttttgcga 6660
aatcaacggt catttttttt gcatcatcgg ttccgcgata gccatcttct attgcatgag 6720
gcggcggtgg cgctgcatcc tgttttaaac cgccctggtc atctgccaac gcataaggca 6780
tgacaagaaa acttgctaat acaatggcct gaaatttcat actaactcct taattgcgtt 6840
tggtttgact tattaagtct ggttgctatt tttataattg ccaaataaga atattgccaa 6900
ttgttataag gcatttaaaa tcagccaact agctgtcaaa tatacagaga atttaactca 6960
ctaaagttaa gaagattgaa aagtcttaaa catattttca gaataatcgg atttatatgt 7020
ttgaaaatta ttatattgga cgagcataca gaaaaagcaa atcaccttta catataaaag 7080
cgtggacaaa aaacagtgaa cattaataga gataaaattg tacaacttgt agataccgat 7140
actattgaaa acctgacatc cgcgttgagt caaagactta tcgcggatca attacgctta 7200
actaccgccg aatcatgcac cggcggtaag ttggctagcg ccctgtgtgc agctgaagat 7260
acacccaaat tttacggtgc aggctttgtt actttcaccg atcaggcaaa gatgaaaatc 7320
ctcagcgtaa gccagcaatc tcttgaacga tattctgcgg tgagtgagaa agtggcagca 7380
gaaatggcaa ccggtgccat agagcgtgcg gatgctgatg tcagtattgc cattaccggc 7440
tacggcggac cggagggcgg tgaagatggt acgccagcgg gtaccgtctg gtttgcgtgg 7500
catattaaag gccagaacta cactgcggtt atgcattttg ctggcgactg cgaaacggta 7560
ttagctttag cggtgaggtt tgccctcgcc cagctgctgc aattactgct ataaccaggc 7620
tggcctggcg atatctcagg ccagccattg gtggtgttta tatgttcaag ccacgatgtt 7680
gcagcatcgg cataatctta ggtgccttac cgcgccattg tcgatacagg cgttccagat 7740
cttcgctgtt acctctggaa aggatcgcct cgcgaaaacg cagcccattt tcacgcgtta 7800
atccgccctg ctcaacaaac cactgataac catcatcggc caacatttgc gtccacagat 7860
aagcgtaata acctgcag 7878

<210> 2
<211> 129
<212> PRT
<213> Escherichia coli

<400> 2
Met Thr Met Ser Arg Arg Asn Thr Asp Ala Ile Thr Ile His Ser Ile
1               5                   10                  15

Leu Asp Trp Ile Glu Asp Asn Leu Glu Ser Pro Leu Ser Leu Glu Lys
            20                  25                  30

Val Ser Glu Arg Ser Gly Tyr Ser Lys Trp His Leu Gln Arg Met Phe

35                  40                  45

Lys Lys Glu Thr Gly His Ser Leu Gly Gln Tyr Ile Arg Ser Arg Lys
    50                  55                  60

Met Thr Glu Ile Ala Gln Lys Leu Lys Glu Ser Asn Glu Pro Ile Leu
65                  70                  75                  80

Tyr Leu Ala Glu Arg Tyr Gly Phe Glu Ser Gln Gln Thr Leu Thr Arg
                85                  90                  95

Thr Phe Lys Asn Tyr Phe Asp Val Pro Pro His Lys Tyr Arg Met Thr
            100                 105                 110

Asn Met Gln Gly Glu Ser Arg Phe Leu His Pro Leu Asn His Tyr Asn
        115                 120                 125

Ser

## Description

### Background of the Invention

Multidrug resistance in bacteria is generally attributed to the acquisition of multiple transposons and plasmids bearing genetic determinants for different mechanisms of resistance (Gold et al. 1996. N. Engl. J. Med. 335:1445). However, descriptions of intrinsic mechanisms that confer multidrug resistance have begun to emerge. The first of these was a chromosomally encoded multiple antibiotic resistance (*mar*) locus in *Escherichia coli (* George and Levy. 1983. J. Bacteriol. 155:531; George and Levy 1983. J. Bacteriol. 155:541). Mar mutants of *E. coli* arose at a frequency of 10⁻⁶ to 10⁻⁷ and were selected by growth on subinhibitory levels of tetracycline or chloramphenicol (George and Levy, supra). These mutants exhibited resistance to tetracyclines, chloramphenicol, penicillins, cephalosporins, puromycin, nalidixic acid, and rifampin (George and Levy, supra). Later, the resistance phenotype was extended to include fluoroquinolones (Cohen et al. 1989. Antimicrob. Agents Chemother. 33:1318), oxidative stress agents ( Ariza et al. 1994. J. Bacteriol. 176:143; Greenberg et al. 1991. J. Bacteriol. 173:4433), and more recently, organic solvents (White et al. 1997. J. of Bacteriology 179:6122; Asako, et al. 1997. J. Bacteriol. 176:143) and household disinfectants, e.g., pine oil and/or triclosan® ( McMurry et al. 1998. FEMS Microbiology Letters 166:305; Moken et al. 1997. Antimicrobial Agents and Chemotherapy 41:2770).

The expression of the Mar phenotype is greater at 30°C than at 37°C (Seoane and Levy. 1995. J. Bacteriol. 177:3414). Continued growth in the same or higher antibiotic concentrations led to increased levels of resistance, thus demonstrating a multiple antibiotic resistance phenotype which could be amplified (George and Levy, supra). Both high- and low-level resistance were decreased or completely reversed by a Tn5 insertion into a single locus at 34 min (1,636.7 kb) on the *E. coli* chromosome, called the *mar* locus. The genetic basis for high-level resistance is only partially attributed to the *mar* locus, since transduction of the locus from high-or low-level *mar* mutants produces only a low level of multidrug resistance.

The *mar* locus consists of two divergently positioned transcriptional units that flank a common promoter/operator region in *E. coli* and *Salmonella typhimurium* (Alekshun and Levy. 1997. Antimicrobial Agents and Chemother. 41: 2067). One operon encodes MarC, a putative integral inner membrane protein without any yet apparent function, but which appears to contribute to the Mar phenotype in some strains. The other operon comprises *marRAB*, encoding the Mar repressor (MarR), which binds *marO* and negatively regulates expression of *marRAB* (Cohen et al. 1994. J. Bacteriol. 175:1484; Martin and Rosner. 1995. Proc. Natl. Acad. Sci. USA 92:5456; Seoane and Levy. 1995. J. Bacterial. 177:530), an activator (MarA), which controls expression of other genes on the chromosome, e.g., the *mar* regulon (Cohen et al. 1994. J. Bacteriol. 175:1484; Gambino et. al. 1993. J. Bacterial. 175:2888; Seoane and Levy. 1995. J. Bacteriol. 177:530), and a putative small protein (MarB) of unknown function.

MarA is a member of the XylS/AraC family of transcriptional activators (Gallegos et al. 1993. Nucleic Acids Res. 21:807). Proteins within this family activate many different genes, some of which produce antibiotic and oxidative stress resistance or control microbial metabolism and virulence (Gallegos et al. supra).

### Summary

The present invention represents an important advance in the battle against drug resistance by elucidating the domains of MarA which are critical in mediating its function. Accordingly, the invention provides, *inter alia,* MarA family protein helix-turn-helix (HTH) domains, mutant MarA family protein helix-turn-helix domains and methods of their use. This new understanding of how MarA family proteins work to activate gene transcription will be invaluable to understanding and ultimately controlling multidrug resistance.

In one aspect, the invention pertains to a method for identifying an antiinfective compound which affects the activity of a MarA family helix-turn-helix domain, by contacting a polypeptide comprising a MarA family helix-turn-helix domain derived from a MarA family protein with a compound under conditions which allow interaction of the compound with the polypeptide such that a complex is formed; and measuring the ability of the compound to affect the activity of a MarA family helix-turn-helix domain as an indication of whether the compound is an antiinfective compound.

In another aspect, the invention pertains to a method for identifying an antiinfective compound which affects the activity of a MarA family helix-turn-helix domain, by contacting a cell expressing a Mar A family helix-turn-helix domain polypeptide derived from a MarA family protein with a compound under conditions which allow interaction of the compound with the polypeptide; and measuring the ability of the compound to affect the activity of a MarA family helix-turn-helix domain polypeptide as an indication of whether the compound is an antiinfective compound.

In one embodiment, the step of measuring the ability of the compound to affect the activity of a MarA family helix-turn-helix domain comprises detecting the ability of the complex to activate transcription from a MarA family member responsive promoter. In a preferred embodiment, the Mar A responsive promoter is selected from the group consisting of *marO, micF*, and *fumC*

In one embodiment, the Mar A responsive promoter is linked to a reporter gene. In a preferred embodiment, the reporter gene is selected from the group consisting of *IacZ, phoA,* or green fluorescence protein.

In one embodiment, the step of measuring comprises measuring the amount of reporter gene product. In another embodiment, the step of measuring comprises measuring the amount of RNA produced by the cell. In yet another embodiment, the step of measuring comprises measuring the amount of a protein produced by the cell. In still another embodiment, the step of measuring comprises using an antibody against a protein produced by the cell.

In another aspect, the invention pertains to a method for identifying an antiinfective compound which affects the activity of a MarA family helix-turn-helix domain, by contacting a polypeptide comprising a Mar A family helix-turn-helix domain derived from a MarA family protein with a compound in a cell-free system under conditions which allow interaction of the compound with the polypeptide such that a complex is formed; and measuring the ability of the compound to affect the activity of a MarA family helix-turn-helix domain as an indication of whether the compound is an antiinfective compound.

In one embodiment, the MarA family helix-turn-helix domain is an isolated polypeptide and the step of measuring the ability of the compound to affect the activity of a MarA family helix-turn-helix domain comprises measuring the ability of the complex to bind to DNA.

In another embodiment of the invention, the method comprises screening a library of bacteriophage displaying on their surface a MarA family helix-turn-helix domain polypeptide, said polypeptide sequence being encoded by a nucleic acid contained within the bacteriophage, for ability to bind a compound to obtain those compounds having affinity for the helix-turn-helix domain, said method by contacting the phage which display the helix-turn-helix domain with a sample of a library of compounds so that the helix-turn-helix domain can interact with and form a complex with any compound having an affinity for the helix-turn-helix domain; contacting the complex of the helix-turn-helix domain and bound compound with an agent that dissociates the bacteriophage from the compound; and identifying the compounds that bound to the helix-turn-helix domain.

In another aspect, the invention pertains to a method for screening a library of bacteriophage displaying on their surface a plurality of polypeptide sequences, each polypeptide sequence being encoded by a nucleic acid contained within the bacteriophage, for ability to bind an immobilized MarA family helix-turn-helix domain, to obtain those polypeptides having affinity for the helix-turn-helix domain, said method by contacting the immobilized helix-turn-helix domain with a sample of the library of bacteriophage so that the helix-turn-helix domain can interact with the different polypeptide sequences and bind those having affinity for the helix-turn-helix domain to form a set of complexes consisting of immobilized helix-turn-helix domain and bound bacteriophage; separating the complexes from bacteriophage which have not formed the complex; contacting the complexes of the helix-turn-helix domain and bound bacteriophage with an agent that dissociates the bound bacteriophage from the complexes; and isolating the dissociated bacteriophage and obtaining the sequence of the nucleic acid encoding the displayed polypeptide, so that amino acid sequences of displayed polypeptides with affinity for helix-turn-helix domain are obtained.

In certain embodiments, the polypeptides of the invention comprise the helix-turn-helix domain most proximal to the carboxy terminus of the MarA family protein from which it is derived. In other embodiments, the polypeptides of the invention comprise the helix-turn-helix domain most proximal to the amino terminus of the MarA family protein from which it is derived. In preferred embodiments, the polypeptides consist of the helix-turn-helix domain most proximal to the carboxy terminus of the MarA family protein from which it is derived. In still other preferred embodiments, the polypeptides consist of the helix-turn-helix domain most proximal to the amino terminus of the MarA family protein from which it is derived.

In preferred embodiments of the invention, the MarA family helix-turn-helix domain is derived from a protein selected from the group consisting of: MarA, RamA, AarP, Rob, SoxS, and PqrA.

In certain embodiments of the invention, a compound identified using the subject methods increases antibiotic susceptibility. In other embodiments, a compound identified using the subject methods reduces infectivity or virulenc of a microbe.

In certain embodiments of the invention, the compound is effective against Gram negative bacteria. In other embodiments, the compound is effective against Gram positive bacteria. In preferred embodiments, the Gram positive bacteria are from a genus selected from the group consisting of: *Enterococcus, Staphylococcus, Clostridium* and *Streptococcus.* In other preferred embodiments, the compound is effective against bacteria from the family *Enterobacteriaceae.* In still other preferred embodiments, the compound is effective against a bacteria of a genus selected from the group consisting of: *Escherichia, Proteus, Klebsiella, Providencia, Enterobacter, Burkholderia, Pseudomonas, Aeromonas, Acinetobacter,* and *Mycobacteria.*

In another aspect, the invention pertains to a cell based method of identifying genetic loci in an microbe which affect antibiotic resistance comprising introducing into said microbe a nucleotide sequence encoding a helix-turn-helix motif of a MarA family protein and assaying for changes in the antibiotic resistance profile of said microbe. In certain embodiments, the invention further comprises assaying for changes in transcription of genetic loci of said microbe. In other embodiments the invention further comprises identifying proteins which are present in different amounts in resistant and susceptible microbes. In other embodiments, the invention further comprising identifying the genes which encode said proteins.

In certain embodiments of the invention the antibiotic to which sensitivity is measured is selected from the group consisting of tetracycline, fluoroquinolones, chloramphenicol, penicillins, cephalosporins, puromycin, nalidixic acid, and rifampin. In other embodiments, the antibiotic is a disinfectant, antiseptic, or surface delivered antibacterial compound. In yet other embodiments, the antibiotic is an antifungal. In still other embodiments, the antibiotic is an antiparasitic.

In another aspect, the invention pertains to a cell-free method of identifying genetic loci in an microbe which affect resistance to antibiotics comprising contacting a nucleic acid molecule of said microbe with a MarA family protein helix-turn-helix domain and allowing complexes to form; separating the nucleic acid molecule which has formed a complex with a helix-turn-helix domain from the helix-turn-helix domain; and identifying the sequence of those nucleic acid molecules which can bind to a MarA family protein helix-turn-helix domain.

In certain embodiments of the invention, the compound to be tested is derived from a library of small molecules. In other embodiments, the compound is a nucleic acid molecule. In still other embodiments, the compound is an antisense or sense oligonucleitide. In yet other embodiments, the compound is a naturally occurring small organic molecule.

In another aspect, the invention pertains to a kit for identifying genetic loci in an microbe which affect resistance to compounds comprising a nucleotide sequence encoding a naturally occurring helix-turn-helix domain of a MarA family protein and mutant, inactive form of a MarA family protein helix-turn-helix domain.

### Brief Description of the Drawings

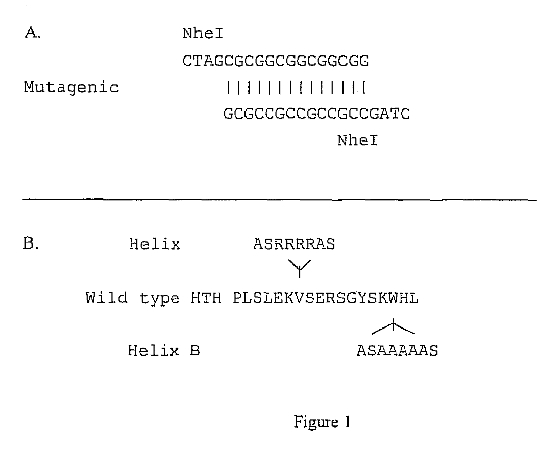

Figure 1 is a schematic showing how the MarA mutants of the present invention were constructed. Panel A shows the nucleotide sequence of the mutagenic oligonucleotide. Panel B shows the wild-type MarA amino acid residues 27 to 44, of MarA (based on the sequence provided in Cohen et al. 1993. J. Bacteriol. 175:1484). Mutants contained the amino acids shown at the indicated sites of insertion in helix A and helix B of the first helix-turn-helix domain (i.e., the most amino terminal helix-turn-helix domain) of MarA. Mutants differ in amino acid composition due to the mutagenic oligonucleotide inserted in opposite orientations.

Figures 2A-2D shows an exemplary alignment of amino acid sequences of selected MarA family protein family members; amino acid sequences that correspond to amino acids 30-76 of MarA are shown in panels A-B and amino acid sequences that correspond to amino acids 77-106 are shown in panels C-D.

Figures 3A-B shows an alignment of amino acid sequences of exemplary MarA family protein family members and MarA family helix-turn--helix domain consensus sequences.

Figure 4 shows exemplary mutagenic oligomers for making mutations in the second helix-turn-helix domain of MarA.

### Detailed Description

The present invention provides an advance in combating drug resistance by identifying the domains of MarA protein family members which mediate resistance, methods of using these domains in drug screening assays to identify compounds which interfere with the mechanism of action of these domains, and methods of identifying other genetic loci which are important in mediating antibiotic resistance in various unrelated bacteria.

Before further description of the invention, certain terms employed in the specification, examples and appended claims are, for convenience, collected here.

### I. Definitions

As used herein, the language "antiinfective compound" includes a compound which reduces the ability of a microbe to produce infection in a host. Antiinfective compounds include those compounds which are static or cidal for microbes, e.g.. an antimicrobial compound which inhibits the proliferation and/or viability of a microbe. Preferred antiinfective compounds increase the susceptibility of microbes to antibiotics or decrease the infectivity or virulence of a microbe. The term "microbe" includes any unicellular microbe, e.g., bacteria, fungi, or protozoa. Therefore, agents which inhibit the proliferation and/or viability of fungi or protozoa are also included in this term. In preferred embodiments, microbes are pathogenic for humans, animals, or plants, however in other embodiments, microbes are involved, e.g., in fouling or spoilage.

As used herein, the term "antibiotic" includes antimicrobial agents isolated from natural sources or chemically synthesized. The term "antibiotic" includes the antimicrobial agents to which the Mar phenotype has been shown to mediate resistance and, as such, includes disinfectants, antiseptics, and surface delivered compounds. For example, any antibiotic, biocide, or other type of antibacterial compound, including agents which induce oxidative stress agents, and organic solvents are included in this term. Preferred antibiotics include: tetracycline, fluoroquinolones, chloramphenicol, penicillins, cephalosporins, puromycin, nalidixic acid, and rifampin.

As used herein, the language "MarA family protein" includes the many naturally occurring transcription regulation proteins which have sequence similarities to MarA and which contain the MarA family signature pattern, which can also be referred to as an XylS/AraC signature pattern. An exemplary signature pattern which defines MarA family proteins is shown, e.g., on PROSITE and is represented by the sequence: [KRQ]-[LIVMA]-X(2)-[GSTALIV]-{FYWPGDN}X(2)-[LIVMSA]-X(4,9)-[LIVMF]-X(2)-[LIVMSTA]-X(2)-[GSTACIL]-X(3)-[GANQRF]-[LIVMFY]-X(4,5)-[LFY]-X(3)-[FYIVA]-{FYWHCM}-X(3)-[GSADENQKR]-X-[NSTAPKL]-[PARL], where X is any amino acid. MarA family proteins have two "helix-turn-helix" domains. This signature pattern was derived from the region that follows the first, most amino terminal, helix-turn-helix domain (HTH1) and includes the totality of the second, most carboxy terminal helix-turn-helix domain (HTH2). (See PROSITE PS00041).

MarA family polypeptide sequences are "structurally related" to one or more known MarA family members, preferably to MarA, This structural relatedness can be shown by sequence similarity between two MarA family polypeptide sequences or between two MarA family nucleotide sequences. Sequence similarity can be shown, e.g., by optimally aligning MarA family member sequences using an alignment program for purposes of comparison and comparing corresponding positions. To determine the degree of similarity between sequences, they will be aligned for optimal comparison purposes (*e*.*g*., gaps may be introduced in the sequence of one protein for nucleic acid molecule for optimal alignment with the other protein or nucleic acid molecules). The amino acid residues or bases and corresponding amino acid positions or bases are then compared. When a position in one sequence is occupied by the same amino acid residue or by the same base as the corresponding position in the other sequence, then the molecules are identical at that position. If amino acid residues are not identical, they may be similar. As used herein, an amino acid residue is "similar" to another amino acid residue if the two amino acid residues are members of the same family of residues having similar side chains. Families of amino acid residues having similar side chains have been defined in the art (see, for example, Altschul et al. 1990. J. Mol. Biol. 215:403) including basic side chains (*e.g*., lysine, arginine, histidine), acidic side chains (*e.g*., aspartic acid, glutamic acid), uncharged polar side chains (*e.g*., glycine, asparagine, glutamine, serine, threonine, tyrosine, cysteine), nonpolar side chains (*e*.*g*., alanine, valine, leucine, isoleucine, proline, phenylalanine, methionine, tryptophan), beta-branched side chains (*e.g*., threonine, valine, isoleucine) and aromatic side chains (*e.g*., tyrosine, phenylalanine, tryptophan). The degree (percentage) of similarity between sequences, therefore, is a function of the number of identical or similar positions shared by two sequences (*i*.*e*., % homology = # of identical or similar positions/total # of positions x 100). Alignment strategies are well known in the art; see, for example, Altschul et al. *supra* for optimal sequence alignment.

MarA family polypeptides share some amino acid sequence similarity with MarA. The nucleic acid and amino acid sequences of MarA as well as other MarA family polypeptides are available in the art. For example, the nucleic acid and amino acid sequence of MarA can be found, e.g., on GeneBank (accession number M96235 or in Cohen et al. 1993. J. Bacteriol. 175:1484, or in SEQ ID NO:1 and SEQ ID NO:2).

The nucleic acid and/or amino acid sequences of MarA can be used as "query sequences" to perform a search against databases (e.g., either public or private) to, for example, identify other MarA family members having related sequences. Such searches can be performed, e.g., using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to MarA family nucleic acid molecules. BLAST protein searches can be performed with the XBLAST program, score = 50, wordlength = 3 to obtain amino acid sequences homologous to MarA protein molecules of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (*e*.*g*., XBLAST and NBLAST) can be used. See http://www.ncbi.nlm.nih.gov.

MarA family members can also be identified as being structurally similiar based on their ability to specifically hybridize to nucleic acid sequences specifying MarA. Such stringent conditions are known to those skilled in the art and can be found e.g., in Current Protocols in Molecular Biology, John Wiley & Sons, N.Y. (1989), 6.3.1-6.3.6. A preferred, non-limiting example of stringent hybridization conditions are hybridization in 6X sodium chloride/sodium citrate (SSC) at about 45°C, followed by one or more washes in 0.2 X SSC, 0.1% SDS at 50-65°C. Conditions for hybridizations are largely dependent on the melting temperature Tm that is observed for half of the molecules of a substantially pure population of a double-stranded nucleic acid. Tm is the temperature in °C at which half the molecules of a given sequence are melted or single-stranded, For nucleic acids of sequence 11 to 23 bases, the Tm can be estimated in degrees C as 2(number of A+T residues) + 4(number of C+G residues). Hybridization or annealing of nucleic acid molecules should be conducted at a temperature lower than the Tm, e.g., 15°C, 20°C, 25°C or 30°C lower than the Tm. The effect of salt concentration (in M of NaCl) can also be calculated, see for example, Brown, A., "Hybridization" pp. 503-506, in The Encyclopedia of Molec. Biol., J. Kendrew, Ed., Blackwell, Oxford (1994).

Preferably, the nucleic acid sequence of a MarA family member identified in this way is at least about 10%, 20%, more preferably at least about 30%, more preferably at least about 40% identical and most preferably at least about 50%, or 60% identical or more with a MarA nucleotide sequence. Preferably, MarA family members have an amino acid sequence at least about 20%, more preferably at least about 30%, more preferably at least about 40% identical and most preferably at least about 50%, or 60% or more identical with a MarA amino acid sequence. However, it will be understood that the level of sequence similarity among microbial regulators of gene transcription, even though members of the same family, is not necessarily high. This is particularly true in the case of divergent genomes where the level of sequence identity may be low, e.g., less than 20% (e.g., *B. burgdorferi* as compared e.g., to *B. subtilis*). Accordingly, structural similarity among MarA family members can also be determined based on "three-dimensional correspondence" of amino acid residues. As used herein, the language "three-dimensional correspondence" is meant to includes residues which spatially correspond, e.g., are in the same functional position of a MarA family protein member as determined, e.g., by x-ray crystallography, but which may not correspond when aligned using a linear alignment program. The language "three-dimensional correspondence" also includes residues which perform the same function, e.g., bind to DNA or bind the same cofactor, as determined, e.g., by mutational analysis.

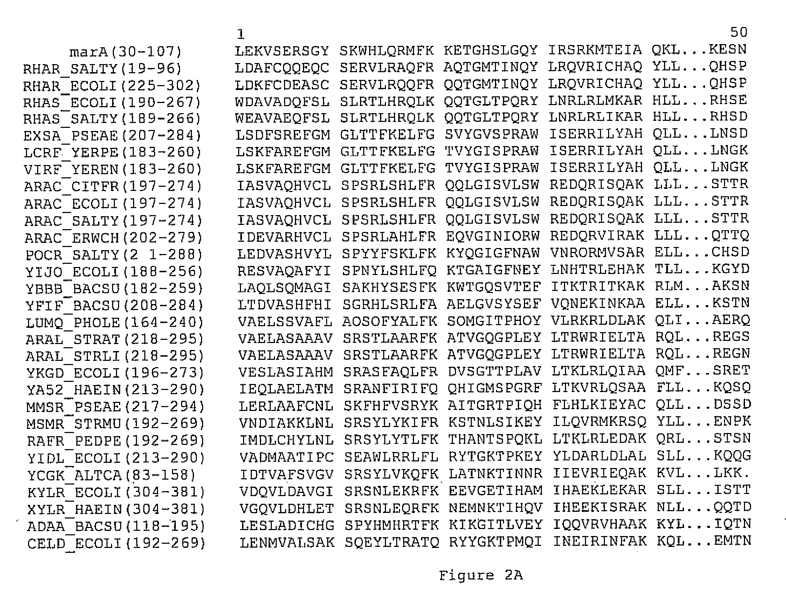

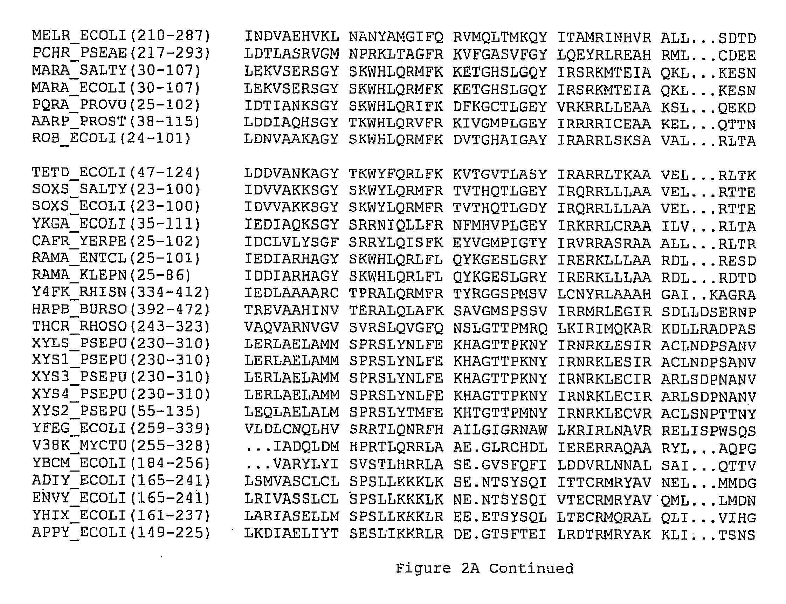

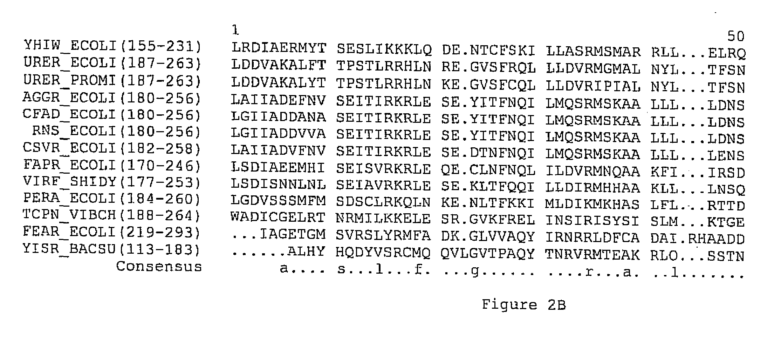

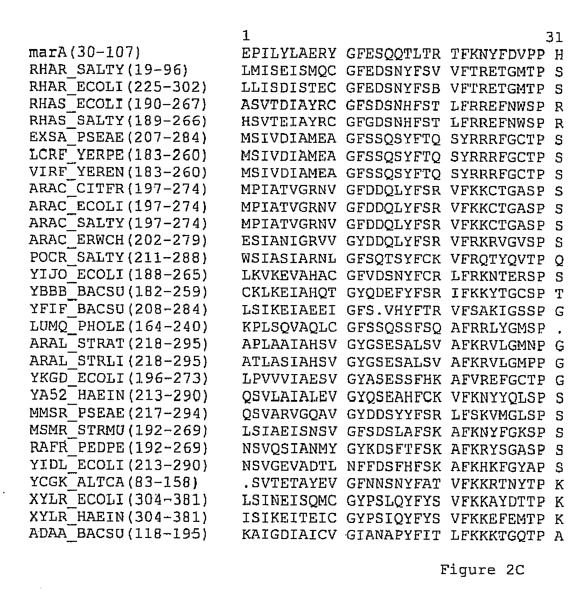

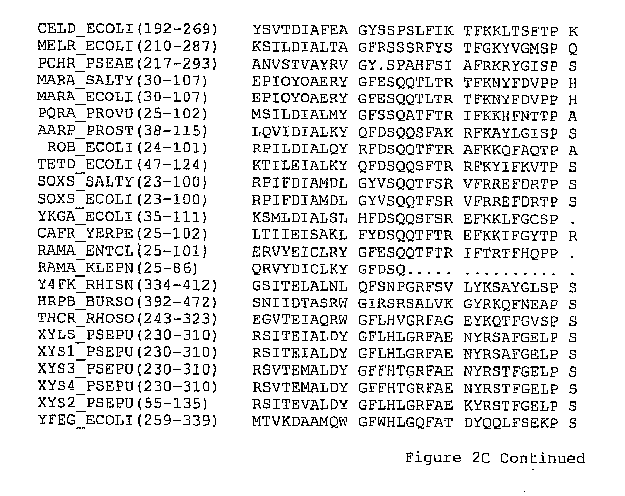

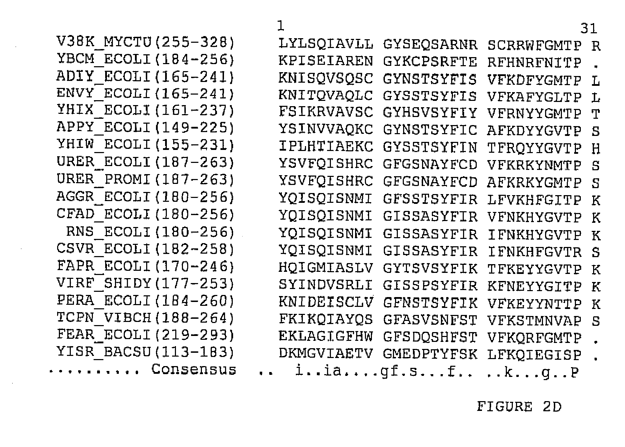

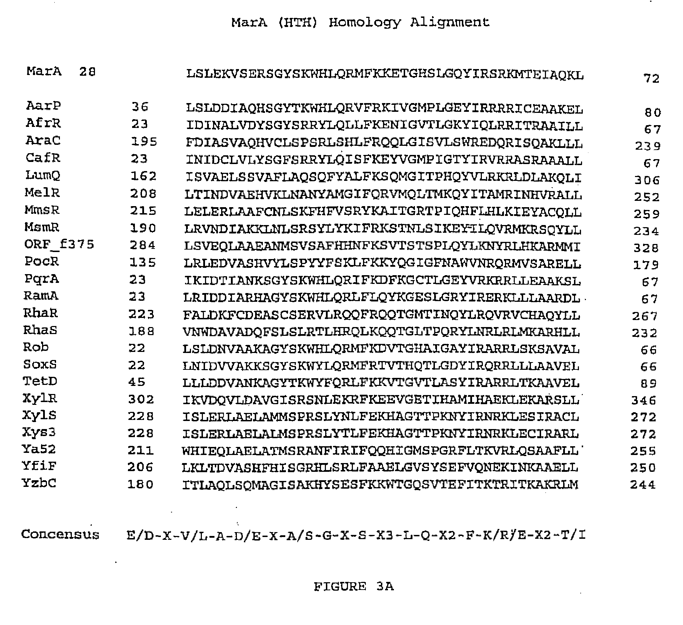

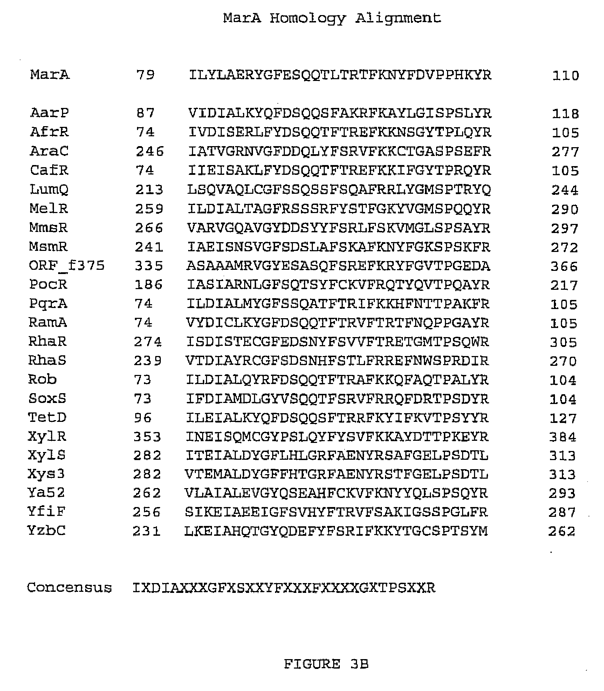

Exemplary MarA family proteins are shown in Table 1, in Figures 2 and 3, and at Prosite (PS00041) and include: AarP, Ada, AdaA, AdiY, AfrR, AggR, AppY, AraC, CafR, CelD, CfaD, CsvR, D90812, EnvY, ExsA, FapR, HrpB, InF, InvF, LcrF, LumQ, MarA, MelR, MixE, MmsR, MsmR, OrfR, Orf_f375, PchR, PerA, PocR, PqrA, RafR, RamA, RhaR, RhaS, Rns, Rob, SoxS, S52856, TetD, TcpN, ThcR, TmbS, U73857, U34257, U21191, UreR, VirF, XyIR, XylS, Xys1, 2, 3, 4, Ya52, YbbB, YfiF, YisR, YzbC, and YijO.

In preferred embodiments, a MarA family protein excludes one or more ofXylS, AraC, and MeIR. In other preferred embodiments, a MarA family protein is involved in antibiotic resistance. In particularly preferred embodiments, a MarA family protein is selected from the group consisting of MarA, RamA, AarP, Rob, SoxS, and PqrA.

Preferred MarA family polypeptides are "naturally occurring." As used herein, a "naturally-occurring" molecule refers to an MarA family molecule having a nucleotide sequence that occurs in nature (*e.g*., encodes a natural MarA family protein). In addition, naturally or non-naturally occurring variants of these polypeptides and nucleic acid molecules which retain the same functional activity, e.g., the ability to bind to DNA and regulate transcription. Such variants can be made, e.g., by mutation using techniques which are known in the art. Alternatively, variants can be chemically synthesized. For example, it will be understood that the MarA family polypeptides described herein, are also meant to include equivalents thereof. Such variants can be made, e.g., by mutation using techniques which are known in the art. Alternatively, variants can be chemically synthesized. For instance, mutant forms of MarA family polypeptides which are functionally equivalent, (e.g., have the ability to bind to DNA and to regulate transcription from an operon) can be made using techniques which are well known in the art. Mutations can include, e.g., at least one of a discrete point mutation which can give rise to a substitution, or by at least one deletion or insertion. For example, random mutagenesis can be used. Mutations can be made by random mutagenesis or using cassette mutagenesis. For the former, the entire coding region of a molecule is mutagenized by one of several methods (chemical, PCR, doped oligonucleotide synthesis) and that collection of randomly mutated molecules is subjected to selection or screening procedures. In the latter, discrete regions of a protein, corresponding either to defined structural or functional determinants (e.g., the first or second helix of a helix-turn-helix domain) are subjected to saturating or semi-random mutagenesis and these mutagenized cassettes are re-introduced into the context of the otherwise wild type allele. In one embodiment, PCR mutagenesis can be used. For example, Megaprimer PCR can be used (O.H. Landt, Gene 96:125-128).

In certain embodiments, such variants have at least 60% amino acid identity with a naturally occurring MarA family member protein. In preferred embodiments, such variants have at least about 70% amino acid identity with a naturally occurring MarA family member protein. In more preferred embodiments, such variants have at least about 80% amino acid identity with a naturally occurring MarA family member protein. In particularly preferred embodiments, such variants have at least about 90% amino acid identity and preferably at least about 95% amino acid identity with a naturally occurring MarA family member protein. In yet other embodiments, a nucleic acid molecule encoding a variant of a MarA family protein is capable of hybridizing under stringent conditions to a nucleic molecule encoding a naturally occurring MarA family protein.

The language "mutant form of a MarA family helix-turn-helix domain" includes mutant forms of such MarA family helix-turn-helix domains which do not retain the same biological activity as the naturally occurring form. For example, such mutants may not bind to a MarA family member promoter or may not initiate transcription from MarA family member responsive promoter or may initiate transcription at a lower level than the naturally occurring MarA family member.

As used herein the language "activity of a MarA family helix-turn-helix domain" includes the ability of the helix-turn-helix domain to interact with DNA, e.g., to bind to a MarA family protein responsive promoter, or to initiate transcription from such a promoter.

As used herein, the language "*marA* family protein responsive promoter" includes promoters which initiate transcription of an operon in a microbe and is structurally or functionally related to the *marA* promoter, e.g., is bound by MarA or a protein related to MarA. Preferably, the *marA* family protein responsive promoter is a *marRAB* promoter. For example, in the *mar* operon, several promoters are *marA* family protein responsive promoters as defined herein, e.g., the 405-bp *ThaI* fragment from the *marO* region is a *marA* family responsive promoter (Cohen et al. 1993. J. Bact. 175:7856). In addition, MarA has been shown to bind to a 16 bp MarA binding site (referred to as the "marbox" within *marO* (Martin et al. 1996. J. Bacteriol. 178:2216). MarA also initiates transcription from the *acrAB; micF; mlr 1,2,3; slp; nfo; inaA; fpr; sodA; soi-17,19; zwf; fumC; or rpsF* promoters (Alekshun and Levy. 1997. Antimicrobial Agents and Chemother. 41:2067). Other *marA* family responsive promoters are known in the art and include: *araBAD, araE, araFGH and araC,* which are activated by AraC; Pm, which is activated by XylS; *melAB* which is activated by MeIR; and *oriC* which is bound by Rob.

The language "MarA family protein responsive promoter" also includes portions of the above promoters which are sufficient to activate transcription upon interaction with a MarA family member protein. The portions of any of the MarA family protein-responsive promoters which are minimally required for their activity can be easily determined by one of ordinary skill in the art, e.g, using mutagenesis. Exemplary techniques are described by Gallegos et al. (1996. J. Bacteriol. 178:6427). A "MarA family protein responsive promoter" also includes non-naturally occurring homologs of MarA family protein responsive promoters which have the same function as naturally occurring MarA family promoters. Preferably such variants have at least 60% nucleotide sequence identity with a naturally occurring MarA family protein responsive promoter. In preferred embodiments, such variants have at least about 70% nucleotide sequence identity with a naturally occurring MarA family protein responsive promoter. In more preferred embodiments, such variants have at least about 80% nucleotide sequence identity with a naturally occurring MarA family protein responsive promoter. In particularly preferred embodiments, such variants have at least about 90% nucleotide sequence identity and preferably at least about 95% nucleotide sequence identity with a naturally occurring MarA family protein responsive promoter. In yet other embodiments nucleic acid molecules encoding variants of MarA family protein responsive promoters are capable of hybridizing under stringent conditions to nucleic acid molecules encoding naturally occurring MarA family protein responsive promoters.

The term "interact" includes close contact between molecules that results in a measurable effect, e.g., the binding of one molecule with another. For example, a MarA family polypeptide can interact with a MarA family protein responsive promoter and alter the level of transcription of DNA. Likewise, compounds can interact with a MarA family polypeptide and alter the activity of a MarA family polypeptide.

As used herein, the term "multiple drug resistance (MDR)" includes resistance to both antibiotic and non-antibiotic compounds. MDR results from the increased transcription of a chromosomal or plasmid encoded genetic locus in an organism, e.g., a *marRAB* locus, that results in the ability of the organism to minimize the toxic effects of a compound to which it has been exposed, as well as to other non-related compounds, e.g., by stimulating an efflux pump(s) or microbiological catabolic or metabolic processes.

As used herein the term "reporter gene" includes any gene which encodes an easily detectable product which is operably linked to a regulatory sequence, e.g.. to a MarA family protein responsive promoter. By operably linked it is meant that under appropriate conditions an RNA polymerase may bind to the promoter of the regulatory region and proceed to transcribe the nucleotide sequence such that the reporter gene is transcribed. In preferred embodiments, a reporter gene consists of the MarA family protein responsive promoter linked in frame to the reporter gene. In certain embodiments, however, it may be desirable to include other sequences, e.g, transcriptional regulatory sequences, in the reporter gene construct. For example, modulation of the activity of the promoter may be effected by altering the RNA polymerase binding to the promoter region, or, alternatively, by interfering with initiation of transcription or elongation of the mRNA. Thus, sequences which are herein collectively referred to as transcriptional regulatory elements or sequences may also be included in the reporter gene construct. In addition, the construct may include sequences of nucleotides that alter translation of the resulting mRNA, thereby altering the amount of reporter gene product.

Examples of reporter genes include, but are not limited to CAT (chloramphenicol acetyl transferase) (Alton and Vapnek (1979), Nature 282: 864-869) luciferase, and other enzyme detection systems, such as beta-galactosidase; firefly luciferase (deWet et al. (1987), Mol. Cell. Biol. 7:725-737); bacterial luciferase (Engebrecht and Silverman (1984), PNAS 1 : 4154-4158; Baldwin et al. (1984), Biochemistry 23: 3663-3667); PhoA, alkaline phosphatase (Toh et al. (1989) Eur. J. Biochem. 182: 231-238, Hall et al. (1983) J. Mol. Appl. Gen. 2:101), human placental secreted alkaline phosphatase (Cullen and Malim (1992) Methods in Enzymol. 216:362-368) and green fluorescent protein (U.S. patent 5,491,084; WO96/23898).

As used herein the term "compound" includes any reagent or test agent which is employed in the assays of the invention and assayed for its utility as an antiinfective compound based on its ability to influence the activity of a MarA family helix-turn-helix domain, e.g., by binding to that domain. More than one compound, e.g., a plurality of compounds, can be tested at the same time for their ability to modulate the activity of a MarA family HTH domain activity in a screening assay.

Compounds that can be tested in the subject assays include antibiotic and non-antibiotic compounds. In one embodiment, compounds include candidate detergent or disinfectant compounds. Exemplary compounds which can be screened for activity include, but are not limited to, peptides, non-peptidic compounds, nucleic acids, carbohydrates, small organic molecules (e.g., polylcetides), and natural product extract libraries. The term "non-peptidic compound" is intended to encompass compounds that are comprised, at least in part, of molecular structures different from naturally-occurring L-amino acid residues linked by natural peptide bonds. However, "non-peptidic compounds" are intended to include compounds composed, in whole or in part, of peptidomimetic structures, such as D-amino acids, non-naturally-occurring L-amino acids, modified peptide backbones and the like, as well as compounds that are composed, in whole or in part, of molecular structures unrelated to naturally-occurring L-amino acid residues linked by natural peptide bonds. "Non-peptidic compounds" also are intended to include natural products.

As used herein, the term "genetic loci" includes an oligonucleotide sequence encoding a peptide or a transcriptional regulatory element (e.g., a promoter, operator, or other regulatory element). A locus may consist of a start codon, a stop codon, and at least one codon encoding an amino acid residue. Typically, a locus is transcribed to produce an mRNA transcript and that transcript is translated to produce a polypeptide.

### II. MarA Family Protein Helix-Turn-Heirx Domains

Helix-turn-helix domains are known in the art and have been implicated in DNA binding (Ann Rev. of Biochem. 1984. 53:293). An example of the consensus sequence for a helix-turn domain can be found in Brunelle and Schleif (1989. J. Mol. Biol. 209:607). The domain has been illustrated by the sequence XXXPhoAlaXXPhoGlyPhoXXXXPhoXXPhoXX, where X is any amino acid and Pho is a hydrophobic amino acid.

The crystal structure of MarA has been determined and the first (most amino terminal) HTH domain of MarA has been identified as comprising from about amino acid 31 to about amino acid 52 and the second HTH domain of MarA has been identified as comprising from about amino acid 79 to about amino acid 102 (Rhee et al. 1998. Proc. Natl. Acad. Sci. USA. 95:10413).

Locations of the helix-turn-helix domains in other MarA family members can easily be found by one of skill in the art. For example using the MarA protein sequence and an alignment program, e.g., the ProDom program, a portion of the MarA amino acid sequence e.g., comprising one or both HTH domains of MarA (such as from about amino acid 30 to about amino acid 107 of MarA as was done to generate Figure 2) to produce an alignment. Exemplary alignments are shown in Figures 2 and 3. Using such an alignment, the amino acid sequences corresponding to the HTH domains of MarA can be identified in other MarA family member proteins. An exemplary consensus sequence for the first helix-turn-helix domain of a MarA family protein can be illustrated as XXXX**A**XXXXX**S**XXX**L**XXX**F**X, where X is any amino acid. An exemplary consensus sequence for the second helix-turn-helix domain of a MarA family protein is illustrated as XX**I**XX**IA**XXX**GF**X**S**XXX**F**XXX[**F**/**Y**], where X is any amino acid. Preferably, a MarA family protein first helix-turn-helix domain comprises the consensus sequence E/D-X-V/L-A-D/E-X-A/S-G-X-S-X3-L-Q-X2-F-K/R/E-X2-T/I. Preferably, a MarA family protein second helix-turn-helix domain comprises the consensus sequence I-X-D-I-A-X3-G-F-X-S-X2-F-X3-F-X4.

Preferably, a MarA family member HTH domain is a MarA HTH domain. The first and second helix-turn-helix domains of MarA are, respectively, EKVSERSGYSKWHLQRMFKKET and ILYLAERYGFESQQTLTRTFKNYF. Other exemplary MarA family helix-turn-helix domains include: about amino acid 210 to about amino acid 229 and about amino acid 259 to about amino acid 278 of MelR; about amino acid 196 to about amino acid 215 and about amino acid 245 to about amino acid 264 of AraC; and about amino acid 230 to about amino acid 249 (or 233-253) and about amino acid 281 to about amino acid 301 (or 282-302) of XylS (see e.g., Brunelle et al. 1989. J. Mol. Biol. 209:607; Niland et al. 1996. J. Mol. Biol. 264:667; Gallegos et al. 1997. Microbiology and Molecular Biology Reviews. 61:393).

"MarA family protein helix-turn-helix domains" are derived from or are homologous to the helix-turn-helix domains found in the MarA family proteins as described supra. In preferred embodiments, a MarA family protein excludes one or more of XylS, AraC, and MelR. In particularly preferred embodiments, a MarA family protein is selected from the group consisting of: MarA, RamA, AarP, Rob, SoxS, and PqrA.

Both of the helix-turn-helix domains present in MarA family proteins are in the carboxy terminal end of the protein. Proteins or portions thereof comprising either or both of these domains can be used in the instant methods. In certain embodiments, a polypeptide which is used in screening for compounds comprises the helix-turn-helix domain most proximal to the carboxy terminus (HTH2) of the MarA family protein from which it is derived. In other embodiments, such a polypeptide comprises the helix-turn-helix domain most proximal to the amino terminus (HTH1) of the MarA family protein from which it is derived. In one embodiment, other polypeptide sequences may also be present, e.g., sequences that might facilitate immobilizing the domain on a support, or, alternatively, might facilitate the purification of the domain.

In preferred embodiments, such a polypeptide consists essentially of the helix-turn-helix domain most proximal to the carboxy terminus of the MarA family protein from which it is derived. In other preferred embodiments, such a polypeptide consists essentially of the helix-turn-helix domain most proximal to the amino terminus of the MarA family protein from which it is derived.

In preferred embodiments, such a polypeptide consists of the helix-turn-helix domain most proximal to the carboxy terminus of the MarA family protein from which it is derived. In other preferred embodiments, such a polypeptide consists of the helix-turn-helix domain most proximal to the amino terminus of the MarA family protein from which it is derived.

MarA family protein helix-turn-helix domains can be made using techniques which are known in the art. The nucleic acid and amino acid sequences of MarA family proteins are available, for example, from GenBank. Using this information, the helix-turn-helix consensus motif and mutational analysis provided herein, one of ordinary skill in the art can identify MarA family helix-turn-helix domains.

In certain embodiments of the invention it will be desirable to obtain "isolated or recombinant" nucleic acid molecules encoding MarA family helix-turn-helix domains or mutant forms thereof. By "isolated or recombinant" is meant a nucleic acid molecule which has been (1) amplified in vitro by, for example, polymerase chain reaction (PCR); (2) recombinantly produced by cloning, or (3) purified, as by cleavage and gel separation; or (4) synthesized by, for example, chemical synthesis. Such a nucleic acid molecule is isolated from the sequences which naturally flank it in the genome and from cellular components.

The isolated or recombinant nucleic acid molecules encoding MarA family helix-turn-helix protein domains can then, for example, be utilized in binding assays, can be expressed in a cell, or can be expressed on the surface of phage, as discussed further below.

In yet other embodiments of the invention, it will be desirable to obtain a substantially purified or recombinant MarA family helix-turn-helix polypeptide. Such polypeptides, for example, can be purified from cells which have been engineered to express an isolated or recombinant nucleic acid molecule which encodes a MarA family helix-turn-helix domain. For example, as described in more detail below, a bacterial cell can be transformed with a plasmid which encodes a MarA family helix-turn-helix domain. The MarA family helix-turn-helix protein can then be purified from the bacterial cells and used, for example, in the cell-free assays described herein.

Purification of a MarA family helix-turn-helix domain can be accomplished using techniques known in the art. For example, column chromatography could be used, or antibodies specific for the domain or for a polypeptide fused to the domain can be employed, for example on a column or in a panning assay.

In preferred embodiments, cells used to express MarA family helix-turn-helix domains for purification, e.g., host cells, comprise a mutation which renders any endogenous MarA family member protein nonfunctional or causes the endogenous protein to not be expressed. In other embodiments, mutations may also be made in MarR or related genes of the host cell, such that repressor proteins which bind to the same promoter as a MarA family protein are not expressed by the host cell.

### III. Mutant MarA Family Helix-Turn-Helix Domains

In certain embodiments of the invention, it will be desirable to use a mutant form of a MarA family protein helix-turn-helix domain, e.g., a non-naturally occurring form of a MarA family helix-turn-helix domain which has altered activity, e.g., does not retain wild type MarA family protein helix-turn-helix domain activity, or which has reduced activity or which is more active when compared to a wild-type MarA family protein helix-turn-helix domain.

Such mutant forms can be made using techniques which are well known in the art. For example, random mutagenesis can be used. Using random mutagenesis one can mutagenize an entire molecule or one can proceed by cassette mutagenesis. In the former instance, the entire coding region of a molecule is mutagenized by one of several methods (chemical, PCR, doped oligonucleotide synthesis) and that collection of randomly mutated molecules is subjected to selection or screening procedures. In the second approach, discrete regions of a protein, corresponding either to defined structural or functional determinants (e.g., the first or second alpha helix of a helix-turn-helix domain) are subjected to saturating or semi-random mutagenesis and these mutagenized cassettes are re-introduced into the context of the otherwise wild type allele.

In a preferred embodiment, PCR mutagenesis is used. For example, Example 2 describes the use of Megaprimer PCR (O.H. Landt, Gene 96:125-128) used to introduce an *Nhe*I restriction site into the centers of both the helix A (position 1989) and helix B (position 2016) regions of the *marA* gene.

In one embodiment, such mutant helix-turn-helix domains comprise one or more mutations in the helix-turn-helix domain most proximal to the carboxy terminus (HTH2) of the MarA family protein molecule. In a preferred embodiment, the mutation comprises an insertion into helix A and helix B of the helix-turn-helix domain most proximal to the carboxy terminus of the MarA family protein. In one embodiment, such mutant helix-turn-helix domains comprise one or more mutations in the helix-turn-helix domain most proximal to the amino terminus (HTH1) of the MarA family protein molecule. In a preferred embodiment, the mutation comprises an insertion into helix A and helix B of the helix-turn-helix domain most proximal to the amino terminus of the MarA family protein. In particularly preferred embodiments, the mutation is selected from the group consisting of: an insertion at an amino acid corresponding to about position 33 of MarA and an insertion at an amino acid position corresponding to about position 42 of MarA. "Corresponding" amino acids can be determined, e.g, using an alignment of the helix-turn-helix domains, such as that shown in Figure 2.

Such mutant forms of MarA family helix-turn-helix motifs are useful as controls to verify the specificity of antiinfective compounds for a MarA family helix-turn-helix domain or as controls for the identification of genetic loci which affect resistance to antiinfectives. For example, the mutant MarA family helix-turn-helix domains described in the appended Examples demonstrate that insertional inactivation of MarA at either helix A or helix B in the first HTH domain abolished the multidrug resistance phenotype in both *E. coli* and *M. smegmatis.* By the use of an assay system such as that described in Example 2, which demonstrates the ability of MarA family protein helix-turn-helix domains to increase antibiotic resistance and that mutant forms of these domains do not have the same effect, one can clearly show that the response of any genetic loci identified is specific to a MarA family helix-turn-helix domain.

### IV. Expression of MarA Family Helix-Turn-Helix Domains

Nucleic acids encoding MarA family protein helix-turn-helix domains can be expressed in cells using vectors. Almost any conventional delivery vector can be used. Such vectors are widely available commercially and it is within the knowledge and discretion of one of ordinary skill in the art to choose a vector which is appropriate for use with a given microbial cell. The sequences encoding these domains can be introduced into a cell on a self-replicating vector or may be introduced into the chromosome of a microbe using homologous recombination or by an insertion element such as a transposon.

These nucleic acids can be introduced into microbial cells using standard techniques, for example, by transformation using calcium chloride or electroporation. Such techniques for the introduction of DNA into microbes are well known in the art.

### V. Methods of Identifying Antimicrobial/Antiinfective Compounds Which Interact With MarA Family Helix-Turn-Helix Domains

In one embodiment, the invention provides for methods of identifying an antiinfective compound which affects the activity of a MarA family helix-turn-helix domain, by contacting a polypeptide comprising a Mar A family helix-turn-helix domain derived from a MarA family protein with a compound under conditions which allow interaction of the compound with the polypeptide. The ability of the compound to reduce an activity of a MarA family protein helix-turn-helix domain is used as an indication of whether the compound is an antimicrobial compound which interferes with the ability of a microbe to grow or an antiinfective compound which interferes with the ability of a microbe to cause infection in a host.

A variety of different techniques can be used to determine whether a compound reduces the activity of a helix-turn-helix domain. For example, the ability of a compound to decrease binding of a MarA family protein to DNA, e.g., to a MarA family protein responsive promoter, or the ability of the compound to reduce MarA family protein initiated transcription from such a promoter can be measured. As described in more detail below, either whole cell or cell free assay systems can be employed.

### A. Whole Cell Assays

In certain embodiments of the invention, the step of determining whether a compound affects the activity of a MarA family helix-turn-helix domain comprises measuring the ability of the compound to reduce the ability of a MarA family helix-turn-helix domain to activate transcription from a MarA responsive promoter. In such an assay, since the MarA family member helix-turn-helix domain would normally bind to the MarA responsive promoter to induce transcription, a compound would be identified based on its ability to reduce this control level of transcription as compared to a cell which had been transfected with the MarA family helix-turn-helix domain but which had not been treated with the compound.

In preferred embodiments, to provide a convenient readout of the transcription from a MarA family protein responsive promoter, such a promoter is linked to a reporter gene. For example, a bacterial cell, e.g., an *E. coli* cell, can be transfected with plasmids comprising a *pm-IacZ* reporter gene construct and a plasmid comprising XylS. XylS activates transcription from the *pm* promoter under control conditions leading to transcription and the production of reporter gene product. The ability of a compound to interfere with this interaction is indicated by a reduction in this control level of transcription and, thus, a reduction in the amount of reporter gene product. The amount of reporter gene product can be measured grossly in intact cells, e.g., by looking at color changes in cells, for example, by using the *lac*Z reporter gene and plating the cells on media supplemented with X-Gal (5-bromo-4-chloro-3-indolyl-β-D-galactopyranoside or, for example, by lysing cells and measuring the amount of product produced, e.g., by absorbance or enzyme activity.

In yet other embodiments, the step of detecting the ability of a compound to induce a change in transcription comprises measuring the amount of RNA produced by the cell. In such embodiments, the cells may or may not comprise a reporter gene construct. For example, the RNA can be isolated from cells which express a MarA family helix-turn-helix domain which have been incubated in the presence and absence of compound. Northern Blots using probes specific for the sequences to be detected can then be performed using techniques known in the art. Sequences which can be detected include any sequences which are linked to a MarA family responsive promoter, including, for example, both endogenous sequences and reporter gene sequences. Exemplary endogenous sequences which can be detected include: *acrAB; micF; mlr 1,2,3; slp; nfo; inaA; fpr; sodA; soi-17,19; zwf; fumC; or rpsF; araBAD, araE, araFGH* and *araC,* which are activated by AraC; *pm*, which is activated by XylS; *melAB* which is activated by MelR; and *oriC* which is activated by Rob., as well as sequences from genetic loci that are identified using the assays described infra.

In yet other embodiments, the ability of a compound to induce a change in transcription from a MarA responsive promoter can be accomplished by measuring the amount of a protein produced by the cell. Proteins which can be detected include any proteins which are produced upon the activation of a MarA family responsive promoter, including, for example, both endogenous sequences and reporter gene sequences. Exemplary endogenous proteins which can be detected include: AcrAB; Mlr 1,2,3; Slp; Nfo; InaA; Fpr; SodA; Soi-17,19; Zwf; FumC; or RpsF promoters (Alekshun and Levy. 1997. Antimicrobial Agents and Chemother, 41:2067). Others are known in the art and include: AraBAD, AraE, AraFGH and AraC, which are activated by AraC; pm, which is activated by XylS; MelAB which is activated by MelR; and oriC which is bound by Rob, as well as proteins translated from genetic loci that are identified using the assays described infra. In one embodiment, a the amount of protein made by a cell can be detected using an antibody against that protein. In other embodiments, the activity of such a protein can be measured.

### B. Cell-Free Assays

In other embodiments, the ability of a compound to affect the activity of a a MarA family helix-turn-helix domain is accomplished using isolated MarA family helix-turn-helix polypeptides in a cell-free system. In such an assay the step of measuring the ability of a compound to affect the activity of the MarA family helix-turn-helix polypeptide is accomplished by measuring the effect of the compound on the ability of helix-turn-helix domain to bind to DNA.

For example, the ability of a helix-turn-helix domain to bind to DNA can be measured by end labeling a nucleic acid molecule which encodes for a MarA family responsive promoter with ³²P using techniques which are known in the art (see e.g., Martin and Rosner. 1995. Proc. Natl. Acad. Sci. USA 92:5456). The helix-turn-helix domain can then be incubated with the compound to be tested to form a complex. The complex can then be incubated with the labeled MarA family protein responsive promoter. The sample can then be electrophoresed to look for changes in the mobility of the sample as compared to the mobility of the helix-turn-helix domain-promoter complex in the absence of the compound (Martin and Rosner, supra).

In yet another method of detecting the ability of a compound to bind a MarA family helix-turn-helix domain, the helix-turn-helix domain polypeptide sequence can be expressed by a bacteriophage. In this embodiment the phages which display the helix-turn-helix domain would then be contacted with a compound so that the helix-turn-helix domain can interact with and potentially form a complex with the compound. Phage which have formed complexes with compounds can then be separated from those which have not. The complex of the helix-turn-helix domain and compound can then be contacted with an agent that dissociates the bacteriophage from the compound. Any compounds that bound to the helix-turn-helix domain can then be isolated and identified.

In a variation of this method that allows for screening of compounds which are polypeptides and which bind to helix-turn-helix domains, a library of bacteriophage which display on their surface a plurality of polypeptide sequences can be tested for their ability to bind a MarA family helix-turn-helix domain to obtain those polypeptides having affinity for the helix-turn-helix domain. The complexes of bound bacteriophage and helix-turn-helix domain can be separated, and then treated with an agent that dissociates the bound bacteriophage from the complexes and the sequence of the nucleic acid encoding the displayed polypeptide can be obtained.

### VII. Microbes Suitable For Testing

Numerous different microbes are suitable for testing in the instant assays. As such, they may be used as intact cells or as sources of DNA as described herein.

In preferred embodiments, microbes for use in the claimed methods are bacteria, either Gram negative or Gram positive bacteria. More specifically, any bacteria that are shown to become resistant to antibiotics, e.g., to display a Mar phenotype are appropriate for use in the claimed methods.

In preferred embodiments, microbes suitable for testing are bacteria from the family *Enterobacteriaceae.* In more preferred embodiments, the antiinfective is effective against a bacteria of a genus selected from the group consisting of: *Escherichia, Proteus, Salmonella, Klebsiella, Providencia, Enterobacter, Burkholderia, Pseudomonas, Aeromonas, Haemophilus, Yersinia, Neisseria,* and *Mycobacteria.*

In yet other embodiments, the microbes to be tested are Gram positive bacteria and are from a genus selected from the group consisting of: *Lactobacillus, Azorhizobium, Streptomyces, Pediococcus, Photobacterium, Bacillus, Enterococcus, Staphylococcus, Clostridium,* and *Streptococcus.*

In other embodiments, the microbes to be tested are fungi. In a preferred embodiment the fungus is from the genus *Mucor* or *Candida,* e.g., *Mucor racmeosus* or *Candida albicans.*

In yet other embodiments, the microbes to be tested are protozoa. In a preferred embdodiment the microbe is a malaria or cryptosporidium parasite.

### VIII. Test Compounds

Compounds for testing in the instant methods can be derived from a variety of different sources and can be known or can be novel. In one embodiment, libraries of compounds are tested in the instant methods to identify MarA family protein blocking agents. In another embodiment, known compounds are tested in the instant methods to identify MarA family protein blocking agents. In a preferred embodiment, compounds among the list of compounds generally regarded as safe (GRAS) by the Environmental Protection Agency are tested in the instant methods.

A recent trend in medicinal chemistry includes the production of mixtures of compounds, referred to as libraries. While the use of libraries of peptides is well established in the art, new techniques have been developed which have allowed the production of mixtures of other compounds, such as benzodiazepines (Bunin et al. 1992. J. Am. Chem. Soc. 114:10987; DeWitt et al. 1993. Proc. Natl. Acad. Sci. USA 90:6909) peptoids (Zuckermann. 1994. J. Med. Chem. 37:2678) oligocarbamates (Cho et al. 1993. Science. 261:1303), and hydantoins (DeWitt et al. supra). Rebek et al. have described an approach for the synthesis of molecular libraries of small organic molecules with a diversity of 104-105 (Carell et al. 1994. Angew. Chem. Int. Ed. Engl. 33:2059; Carell et al. Angew. Chem. Int. Ed. Engl. 1994. 33:2061).

The compounds of the present invention can be obtained using any of the numerous approaches in combinatorial library methods known in the art, including: biological libraries; spatially addressable parallel solid phase or solution phase libraries, synthetic library methods requiring deconvolution, the 'one-bead one-compound' library method, and synthetic library methods using affinity chromatography selection. The biological library approach is limited to peptide libraries, while the other four approaches are applicable to peptide, non-peptide oligomer or small molecule libraries of compounds (Lam, K.S. Anticancer Drug Des. 1997. 12:145).

Exemplary compounds which can be screened for activity include, but are not limited to, peptides, nucleic acids, carbohydrates, small organic molecules, and natural product extract libraries. In one embodiment, the test compound is a peptide or peptidomimetic. In another, preferred embodiment, the compounds are small, organic non-peptidic compounds.

Other exemplary methods for the synthesis of molecular libraries can be found in the art, for example in: Erb et al. 1994. Proc. Natl. Acad. Sci. USA 91:11422; Horwell et al. 1996 Immunopharmacology 33:68; and in Gallop et al. 1994. J. Med. Chem. 37:1233.

Libraries of compounds may be presented in solution (e.g., Houghten (1992) Biotechniques 13:412-421), or on beads (Lam (1991) Nature 354:82-84), chips (Fodor (1993) Nature 364:555-556), bacteria (Ladner USP 5,223,409), spores (Ladner USP '409), plasmids (Cull et al. (1992) Proc Natl Acad Sci USA 89:1865-1869) or on phage (Scott and Smith (1990) Science 249:386-390); (Devlin (1990) Science 249:404-406); (Cwirla et al. (1990) Proc. Natl. Acad. Sci. 87:6378-6382); (Felici (1991) J. Mol. Biol. 222:301-310); (Ladner *supra*.)*.* Other types of peptide libraries may also be expressed, see, for example, U.S. Patents 5,270,181 and 5,292,646). In still another embodiment, combinatorial polypeptides can be produced from a cDNA library.

In other embodiments, the compounds can be nucleic acid molecules. In preferred embodiments, nucleic acid molecules for testing are small oligonucleotides. Such oligonucleotides can be randomly generated libraries of oligonucleotides or can be specifically designed to reduce the activity of a MarA protein family member helix-turn-helix domain. For example, in one embodiment, these oligonucleotides are sense or antisense oligonucleotides. In preferred embodiments, oligonucleotides for testing are sense to the binding site of a MazA protein family member helix-turn-helix domain. Methods of designing such oligonucleotides given the sequences of the MarA family member protein helix-turn-helix domains is within the skill of the art.

In preferred embodiments, controls should be included to ensure that any compounds which are identified using the subject assays do not merely appear to decrease the activity of a MarA family helix-turn-helix domain because they inhibit protein synthesis. For example, if a compound appears to inhibit the synthesis of a protein being translated from RNA which is transcribed upon activation of a MarA family responsive promoter, it may be desirable to show that the synthesis of a control, e.g., a protein which is being translated from RNA which is not transcribed upon activation of a MarA family responsive promoter, is not affected by the addition of the same compound. For example, the amount of the MarA family helix-turn-helix polypeptide being made or the amount of an endogenous protein could be tested. In another embodiment the microbe could be transformed with another plasmid comprising a promoter which is not a MarA family responsive promoter and a protein operably linked to that promoter. The expression of this protein could be used to normalize the amount of protein produced in the presence and absence of compound.

### X. Methods of Identifying Genetic Loci in an Microbe Which Affect Resistance

A variety of different techniques can be used to identify new genetic loci which are involved in mediating antibiotic resistance. For example, either whole cell or cell free assay systems can be employed utilizing at least one MarA family helix-turn-helix domain.

### A. Cell-Based Assays

In one embodiment the invention provides cell based method of identifying genetic loci in an microbe which affect resistance to antibiotics. In such an assay a nucleotide sequence encoding a helix-turn-helix motif of a MarA family protein is introduced into a microbe and the microbe is tested for changes in its antibiotic resistance profile, for example, by monitoring changes in growth in media containing antibiotics or by detecting a reduction in the zone of inhibition around an antibiotic disc. The ability of a MarA family protein helix-turn-helix domain to decrease antibiotic sensitivity is an indication that the microbe comprises a MarA family protein responsive endogenous genetic loci which are involved in mediating antibiotic resistance.

In another embodiment, the above method can further involve assaying for changes in transcription of the genetic loci identified in the microbe. For example, a test microbe can be transformed with a vector bearing a MarA family helix-turn-helix domain. Suitable control microbes include those which lack any such heterologous DNA or are transformed with a vector bearing a mutant form of a MarA family helix-turn-helix domain. The total RNA from the test and control microbes can be isolated. This can be done, for example, by making a cDNA library from both of the strains. The cDNAs from the test and control strains can then be incubated together under conditions which are favorable to hybridization. cDNAs which do not hybridize and remain single stranded may be involved in mediating antibiotic resistance and can be isolated and sequenced using standard techniques.

In another example of a method by which the total mRNA from cells bearing a MarA family helix-turn-helix domain can be compared to cells with lack such a domain or bear a mutant form of such a domain, a cDNA library can be made from the total RNA of these cells. This cDNA library can be used to generate labeled probes which can be used in a standard Northern blot screen. Any cDNA probes that hybridize to the mRNA of the cells comprising a MarA family helix-turn-helix domain, but not to the mRNA from control cells will be involved in mediating antibiotic resistance. Once these cDNA probes which specifically hybridize to cells comprising a MarA family helix-turn-helix domain are identified, these probes can be used to identify genes involved in mediating antibiotic resistance using standard techniques.

### A. Cell-Free Assays

In other embodiments of the invention, MarA family protein responsive genetic loci involved in mediating antibiotic resistance are identified using cell-free assays. In one embodiment, a cell-free method of identifying such genetic loci involves contacting a nucleic acid molecule of the microbe with a MarA family protein helix-turn-helix domain and allowing complexes to form. The helix-turn-helix domain-nucleic acid molecule complexes are separated from the uncomplexed helix-turn-helix domains and the sequence of those nucleic acid molecules which can bind to a MarA family protein helix-turn-helix domain can then be sequenced to identify loci involved in mediating antibiotic resistance.

For example, substantially purified MarA family protein helix-turn-helix domain polypeptide is mixed with the fragmented genomic DNA of an microbe under conditions which permit the polypeptide to bind to appropriate DNA sequences. DNA fragments to which the helix-turn-helix domain has bound can be isolated using a column, filters, polyacrylamide gels, or any other methods well known to those of ordinary skill in the art. The DNA which has bound to the helix-turn-helix domain can then be released from the domain and cloned into vectors or used as probes to locate and isolate the genes to which they correspond. Any such genes can then be sequenced.

In another aspect the invention also provides for kits for identifying genetic loci in an microbe which affect resistance to compounds. Such kits comprise a nucleotide sequence encoding a MarA family protein helix-turn-helix domain and/or substantially purified MarA family protein helix-turn-helix domains and nucleotide sequence encoding a mutant form of a MarA family protein helix-turn-helix domain and/or substantially purified mutant forms of a MarA family protein helix-turn-helix domain. By providing both functional MarA family protein helix-turn-helix domains and mutant forms of such domains, the subject kits provide both the test and control reagents which facilitate both optimal performance of the claimed methods and optimal interpretation of results.

### XI. Formulations Comprising Compounds Identified in the Instant Assays

The invention provides pharmaceutically acceptable compositions which include a therapeutically-effective amount or dose of a compound identified in any of the instant assays and one or more pharmaceutically acceptable carriers (additives) and/or diluents. A composition can also include a second antimicrobial agent, e.g., an antibiotic.

As described in detail below, the pharmaceutical compositions can be formulated for administration in solid or liquid form, including those adapted for the following: (1) oral administration, for example, aqueous or non-aqueous solutions or suspensions, tablets, boluses, powders, granules, pastes; (2) parenteral administration, for example, by subcutaneous, intramuscular or intravenous injection as, for example, a sterile solution or suspension; (3) topical application, for example, as a cream, ointment or spray applied to the skin; (4) intravaginally or intrarectally, for example, as a pessary, cream, foam, or suppository; or (5) aerosol, for example, as an aqueous aerosol, liposomal preparation or solid particles containing the compound.

The phrase "pharmaceutically-acceptable carrier" as used herein means a pharmaceutically-acceptable material, composition or vehicle, such as a liquid or solid filler, diluent, excipient, solvent or encapsulating material, involved in carrying or transporting the antiinfective agents or compounds of the invention from one organ, or portion of the body, to another organ, or portion of the body without affecting its biological effect. Each carrier should be "acceptable" in the sense of being compatible with the other ingredients of the composition and not injurious to the subject. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical compositions. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microbes may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Pharmaceutical compositions of the present invention may be administered to epithelial surfaces of the body orally, parenterally, topically, rectally, nasally, intravaginally, intracisternally. They are of course given by forms suitable for each administration route. For example, they are administered in tablets or capsule form, by injection, inhalation, eye lotion, ointment, etc., administration by injection, infusion or inhalation; topical by lotion or ointment; and rectal or vaginal suppositories.

The phrases "parenteral administration" and "administered parenterally" as used herein mean modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systemically," "peripheral administration" and "administered peripherally" as used herein mean the administration of a sucrose octasulfate and/or an antibacterial, drug or other material other than directly into the central nervous system, such that it enters the subject's system and, thus, is subject to metabolism and other like processes, for example, subcutaneous administration.

In some methods, the compositions of the invention can be topically administered to any epithelial surface. An "epithelial surface" according to this invention is defined as an area of tissue that covers external surfaces of a body, or which lines hollow structures including, but not limited to, cutaneous and mucosal surfaces. Such epithelial surfaces include oral, pharyngeal, esophageal, pulmonary, ocular, aural, nasal, buccal, lingual, vaginal, cervical, genitourinary, alimentary, and anorectal surfaces.

Compositions can be formulated in a variety of conventional forms employed for topical administration. These include, for example, semi-solid and liquid dosage forms, such as liquid solutions or suspensions, suppositories, douches, enemas, gels. creams, emulsions, lotions, slurries, powders, sprays, lipsticks, foams, pastes, toothpastes, ointments, salves, balms, douches, drops, troches, chewing gums, lozenges, mouthwashes, rinses.

Conventionally used carriers for topical applications include pectin, gelatin and derivatives thereof, polylactic acid or polyglycolic acid polymers or copolymers thereof, cellulose derivatives such as methyl cellulose, carboxymethyl cellulose, or oxidized cellulose, guar gum, acacia gum, karaya gum, tragacanth gum, bentonite, agar, carbomer, bladderwrack, ceratonia, dextran and derivatives thereof, ghatti gum, hectorite, ispaghula husk, polyvinypyrrolidone, silica and derivatives thereof, xanthan gum, kaolin, talc, starch and derivatives thereof, paraffin, water, vegetable and animal oils, polyethylene, polyethylene oxide, polyethylene glycol, polypropylene glycol, glycerol, ethanol, propanol, propylene glycol (glycols, alcohols), fixed oils, sodium, potassium, aluminum, magnesium or calcium salts (such as chloride, carbonate, bicarbonate, citrate, gluconate, lactate, acetate, gluceptate or tartrate).

Such compositions can be particularly useful, for example, for treatment or prevention of an unwanted cell, e.g., vaginal *Neisseria gonorrhoeae*, or infections of the oral cavity, including cold sores, infections of eye, the skin, or the lower intestinal tract. Standard composition strategies for topical agents can be applied to the antiinfective compounds or a pharmaceutically acceptable salt thereof in order to enhance the persistence and residence time of the drug, and to improve the prophylactic efficacy achieved.

For topical application to be used in the lower intestinal tract or vaginally, a rectal suppository, a suitable enema, a gel, an ointment, a solution, a suspension or an insert can be used. Topical transdermal patches may also be used. Transdermal patches have the added advantage of providing controlled delivery of the compositions of the invention to the body. Such dosage forms can be made by dissolving or dispersing the agent in the proper medium.

Compositions of the invention can be administered in the form of suppositories for rectal or vaginal administration. These can be prepared by mixing the agent with a suitable non-irritating carrier which is solid at room temperature but liquid at rectal temperature and therefore will melt in the rectum or vagina to release the drug. Such materials include cocoa butter, beeswax, polyethylene glycols, a suppository wax or a salicylate, and which is solid at room temperature, but liquid at body temperature and, therefore, will melt in the rectum or vaginal cavity and release the active agent.

Compositions which are suitable for vaginal administration also include pessaries, tampons, creams, gels, pastes, foams, films, or spray compositions containing such carriers as are known in the art to be appropriate. The carrier employed in the sucrose octasulfate /contraceptive agent should be compatible with vaginal administration and/or coating of contraceptive devices. Combinations can be in solid, semi-solid and liquid dosage forms, such as diaphragm, jelly, douches, foams, films, ointments, creams, balms, gels, salves, pastes, slurries, vaginal suppositories; sexual lubricants, and coatings for devices, such as condoms, contraceptive sponges, cervical caps and diaphragms.

For ophthalmic applications, the pharmaceutical compositions can be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the compositions can be formulated in an ointment such as petrolatum. Exemplary ophthalmic compositions include eye ointments, powders, solutions and the like.

Powders and sprays can contain, in addition to sucrose octasulfate and/or antibiotic or contraceptive agent(s), carriers such as lactose, talc, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Ordinarily, an aqueous aerosol is made by formulating an aqueous solution or suspension of the agent together with conventional pharmaceutically acceptable carriers and stabilizers. The carriers and stabilizers vary with the requirements of the particular compound, but typically include nonionic surfactants (Tweens, Pluronics, or polyethylene glycol), proteins like serum albumin, sorbitan esters, oleic acid, lecithin, amino acids such as glycine, buffers, salts, sugars or sugar alcohols. Aerosols generally are prepared from isotonic solutions.

Compositions of the invention can also be orally administered in any orally-acceptable dosage form including, but not limited to, capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of sucrose octasulfate and/or antibiotic or contraceptive agent(s) as an active ingredient. A compound may also be administered as a bolus, electuary or paste. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added.

Tablets, and other solid dosage forms, such as dragees, capsules, pills and granules, may be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1.3-butylene glycol, oils (in particular, cottonseed, groundnut, com, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the antiinfective agent(s) may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Sterile injectable forms of the compositions of this invention can be aqueous or oleaginous suspension. These suspensions may be formulated according to techniques known in the art using suitable dispersing or wetting agents and suspending agents. Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

The sterile injectable preparation may also be a sterile injectable solution or suspension in a nontoxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as Ph. Helv or similar alcohol.

The antiinfective agent or a pharmaceutically acceptable salt thereof will represent some percentage of the total dose in other dosage forms in a material forming a combination product, including liquid solutions or suspensions, suppositories, douches, enemas, gels, creams, emulsions, lotions slurries, soaps, shampoos, detergents, powders, sprays, lipsticks, foams, pastes, toothpastes, ointments, salves, balms, douches, drops, troches, lozenges, mouthwashes, rinses and others. Creams and gels for example, are typically limited by the physical chemical properties of the delivery medium to concentrations less than 20% (e.g., 200 mg/gm). For special uses, far less concentrated preparations can be prepared, (e.g., lower percent formulations for pediatric applications). For example, the pharmaceutical composition of the invention can comprise sucrose octasulfate in an amount of 0.001-99%, typically 0.01-75%, more typically 0.1-20%, especially 1-10% by weight of the total preparation. In particular, a preferred concentration thereof in the preparation is 0.5-50%, especially 0.5-25%, such as 1-10%. It can be suitably applied 1-10 times a day, depending on the type and severity of the condition to be treated or prevented.

Given the low toxicity of an antiinfective agent or a pharmaceutically acceptable salt thereof over many decades of clinical use as an anti-ulcerant [W.R. Garnett, Clin. Pharm. 1:307-314 (1982); R.N. Brogden et al., Drugs 27:194-209 (1984); D.M. McCarthy, New Eng J Med., 325:1017-1025 (1991), an upper limit for the therapeutically effective dose is not a critical issue.

For prophylactic applications, the pharmaceutical composition of the invention can be applied prior to potential infection. The timing of application prior to potential infection can be optimized to maximize the prophylactic effectiveness of the compound. The timing of application will vary depending on the mode of administration, the epithelial surface to which it is applied, the surface area, doses, the stability and effectiveness of composition under the pH of the epithelial surface, the frequency of application, e.g., single application or multiple applications. One skilled in the art will be able to determine the most appropriate time interval required to maximize prophylactic effectiveness of the compound.

The practice of the present invention will employ, unless otherwise indicated, conventional techniques of cell biology, cell culture, molecular biology, microbiology, recombinant DNA, and immunology, which are within the skill of the art. Such techniques are explained fully in the literature. See, for example, Genetics; Molecular Cloning A Laboratory Manual, 2nd Ed., ed. by Sambrook, J. et al. (Cold Spring Harbor Laboratory Press (1989)); Short Protocols in Molecular Biology, 3rd Ed., ed. by Ausubel, F. et al. (Wiley, NY (1995)); DNA Cloning, Volumes I and II (D. N. Glover ed., 1985); Oligonucleotide Synthesis (M. J. Gait ed. (11984)); Mullis et al. U.S. Patent No: 4,683,195; Nucleic Acid Hybridization (B. D. Hames & S. J. Higgins eds. (11984)); the treatise, Methods In Enzymology (Academic Press, Inc., N.Y.); Immunochemical Methods In Cell And Molecular Biology (Mayer and Walker, eds., Academic Press, London (1987)); Handbook Of Experimental Immunology, Volumes I-IV (D. M. Weir and C. C. Blackwell, eds. (1986)); and Miller, J. Experiments in Molecular Genetics (Cold Spring Harbor Press, Cold Spring Harbor, N.Y. (1972)).

The contents of all references, pending patent applications and published patents, cited throughout this application are hereby expressly incorporated by reference. In addition, the contents of U.S. patent 5,650,321 are also expressly incorporated by this reference.

For the avoidance of doubt, the present application is directed to the subject-matter described in the following numbered paragraphs:
1. A method for identifying an antiinfective compound which affects the activity of a MarA family helix-turn-helix domain, comprising: contacting a polypeptide comprising a Mar A family helix-turn-helix domain derived from a MarA family protein with a compound under conditions which allow interaction of the compound with the polypeptide such that a complex is formed; and measuring the ability of the compound to affect the activity of a MarA family helix-turn-helix domain as an indication of whether the compound is an antiinfective compound.
2. A method for identifying an antiinfective compound which affects the activity of a MarA family helix-turn-helix domain, comprising: contacting a cell expressing a Mar A family helix-turn-helix domain polypeptide derived from a MarA family protein with a compound under conditions which allow interaction of the compound with the polypeptide; and measuring the ability of the compound to affect the activity of a MarA family helix-turn-helix domain polypeptide as an indication of whether the compound is an antiinfective compound.
3. The method of paragraph 2, wherein the step of measuring the ability of the compound to affect the activity of a MarA family helix-turn-helix domain comprises detecting the ability of the complex to activate transcription from a MarA family member responsive promoter.
4. The method of paragraph 3, wherein the Mar A responsive promoter is selected from the group consisting of *marO, micF, and fumC*.
5. The method of paragraph 3, wherein the Mar A responsive promoter is linked to a reporter gene.
6. The method of paragraph 5, wherein the reporter gene is selected from the group consisting of lacZ, phoA, or green fluorescence protein.
7. The method of paragraph 5, wherein the step of measuring comprises measuring the amount of reporter gene product.
8. The method of paragraph 3, wherein the step of measuring comprises measuring the amount of RNA produced by the cell.
9. The method of paragraph 3, wherein the step of measuring comprises measuring the amount of a protein produced by the cell.
10. The method of paragraph 9, wherein the step of measuring comprises using an antibody against a protein produced by the cell.
11. A method for identifying an antiinfective compound which affects the activity of a MarA family helix-turn-helix domain, comprising: contacting a polypeptide comprising a Mar A family helix-turn-helix domain derived from a MarA family protein with a compound in a cell-free system under conditions which allow interaction of the compound with the polypeptide such that a complex is formed; and measuring the ability of the compound to affect the activity of a MarA family helix-turn-helix domain as an indication of whether the compound is an antiinfective compound.
12. The method of paragraph 11, wherein the MarA family helix-turn-helix domain is an isolated polypeptide and the step of measuring the ability of the compound to affect the activity of a MarA family helix-turn-helix domain comprises measuring the ability of the complex to bind to DNA.
13. The method of paragraph 11, wherein said method comprises screening a library of bacteriophage displaying on their surface a MarA family helix-turn-helix domain polypeptide, said polypeptide sequence being encoded by a nucleic acid contained within the bacteriophage, for ability to bind a compound to obtain those compounds having affinity for the helix-turn-helix domain, said method comprising:
   contacting the phage which display the helix-turn-helix domain with a sample of a library of compounds so that the helix-turn-helix domain can interact with and form a complex with any compound having an affinity for the helix-turn-helix domain;
   contacting the complex of the helix-turn-helix domain and bound compound with an agent that dissociates the bacteriophage from the compound; and identifying the compounds that bound to the helix-turn-helix domain.
14. A method for screening a library of bacteriophage displaying on their surface a plurality of polypeptide sequences, each polypeptide sequence being encoded by a nucleic acid contained within the bacteriophage, for ability to bind an immobilized MarA family helix-turn-helix domain, to obtain those polypeptides having affinity for the helix-turn-helix domain, said method comprising contacting the immobilized helix-turn-helix domain with a sample of the library of bacteriophage so that the helix-turn-helix domain can interact with the different polypeptide sequences and bind those having affinity for the helix-turn-helix domain to form a set of complexes consisting of immobilized helix-turn-helix domain and bound bacteriophage; separating the complexes from bacteriophage which have not formed the complex; contacting the complexes of the helix-turn-helix domain and bound bacteriophage with an agent that dissociates the bound bacteriophage from the complexes; and isolating the dissociated bacteriophage and obtaining the sequence of the nucleic acid encoding the displayed polypeptide, so that amino acid sequences of displayed polypeptides with affinity for helix-turn-helix domain are obtained.
15. The method of paragraph 1, wherein said polypeptide comprises the helix-turn-helix domain most proximal to the carboxy terminus of the MarA family protein from which it is derived.
16. The method of paragraph 1, wherein said polypeptide comprises the helix-turn-helix domain most proximal to the amino terminus of the MarA family protein from which it is derived.
17. The method of paragraph 1, wherein said polypeptide consists of the helix-turn-helix domain most proximal to the carboxy terminus of the MarA family protein from which it is derived.
18. The method of paragraph 1, wherein said polypeptide consists of the helix-turn-helix domain most proximal to the amino terminus of the MarA family protein from which it is derived.
19. The method of paragraph 1, wherein the MarA family helix-turn-helix domain is derived from a protein selected from the group consisting of: MarA, RamA, AarP, Rob, SoxS, and PqrA.
20. The method of paragraph 1, wherein the compound increases antibiotic susceptibility.
21. The method of paragraph 1, wherein the compound reduces infectivity or virulence.
22. The method ofparagraph 1, wherein the compound is effective against Gram negative bacteria.
23. The method of paragraph 1, wherein the compound is effective against Gram positive bacteria.
24. The method of paragraph 23, wherein the Gram positive bacteria are from a genus selected from the group consisting of: *Enterococcus, Staphylococcus, Clostridium and Streptococcus*.
25. The method of paragraph 1, wherein the compound is effective against bacteria from the family *Enterobacteriaceae*.
26. The method of paragraph 1, wherein the compound is effective against a bacteria of a genus selected from the group consisting of: *Escherichia, Proteus, Klebsiella, Providencia, Enterobacter, Burkholderia, Pseudomonas, Aeromonas, Acinetobacter and Mycobacteria*.
27. A cell based method of identifying genetic loci in an microbe which affect antibiotic resistance comprising introducing into said microbe a nucleotide sequence encoding a helix-turn-helix motif of a MarA family protein and assaying for changes in the antibiotic resistance profile of said microbe.
28. The method of paragraph 27, further comprising assaying for changes in transcription of genetic loci of said microbe.
29. The method of paragraph 28, further comprising identifying proteins which are present in different amounts in resistant and susceptible microbes.
30. The method of paragraph 29, further comprising identifying the genes which encode said proteins.
31. The method of paragraph 27, wherein said polypeptide comprises the helix-turn-helix domain most proximal to the carboxy terminus of the MarA family protein from which it is derived.
32. The method of paragraph 27, wherein said polypeptide comprises the helix-turn-helix domain most proximal to the amino terminus of the MarA family protein from which it is derived.
33. The method of paragraph 27, wherein said polypeptide consists of the helix-turn-helix domain most proximal to the carboxy terminal end of the MarA family protein from which it is derived.
34. The method of paragraph 27, wherein said polypeptide consists of the helix-furn-helix domain most proximal to the amino terminal end of the MarA family protein from which it is derived.
35. The method of paragraph 27, wherein the MarA family helix-turn-helix domain is derived from a protein selected from the group consisting of: MarA, RamA, AarP, Rob, SoxS, and PqrA.
36. The method of paragraph 27, wherein said microbe is a bacterium.
37. The method of paragraph 20, wherein the antibiotic is selected from the group consisting of: tetracycline, fluoroquinolones, chloramphenicol, penicillins, cephalosporins, puromycin, nalidixic acid, and rifampin.
38. The method of paragraph 20, wherein the antibiotic is a disinfectant, antiseptic, or surface delivered antibacterial compound.
39. The method of paragraph 20, wherein the antibiotic is an antifungal.
40. The method of paragraph 20, wherein the antibiotic is an antiparasitic.
41. A cell-free method of identifying genetic loci in an microbe which affect resistance to antibiotics comprising contacting a nucleic acid molecule of said microbe with a MarA family protein helix-turn-helix domain and allowing complexes to form; separating the nucleic acid molecule which has formed a complex with a helix-turn-helix domain from the helix-turn-helix domain; and identifying the sequence of those nucleic acid molecules which can bind to a MarA family protein helix-turn-helix domain.
42. The method of paragraph 40, wherein said polypeptide comprises the helix-turn-helix domain most proximal to the carboxy terminus of the MarA family protein from which it is derived.
43. The method of paragraph 40, wherein said polypeptide comprises the helix-turn-helix domain most proximal to the amino terminus of the MarA family protein from which it is derived.
44. The method of paragraph 40, wherein said polypeptide consists of the helix-turn-helix domain most proximal to the carboxy terminal end of the MarA family protein from which it is derived.
45. The method of paragraph 40, wherein said polypeptide consists of the helix-turn-helix domain most proximal to the amino terminal end of the MarA family protein from which it is derived.
46. The method of paragraph 40, wherein the MarA family helix-turn-helix domain is derived from a protein selected from the group consisting of: MarA, RamA, AarP, Rob, SoxS, and PqrA.
47. The method of paragraph 40, wherein said microbe is a bacterium.
48. The method of paragraph 1, wherein the compound is derived from a library of small molecules.
49. The method of paragraph 1, wherein the compound is a nucleic acid molecule.
50. The method of paragraph 49, wherein the compound is an antisense or sense oligonucleitide.
51. The method of paragraph 1, wherein the compound is a naturally occurring small organic molecule.
52. An isolated or recombinant mutant MarA family helix-turn-helix domain comprising a mutation in the helix-turn-helix domain most proximal to the carboxy terminus of the MarA family protein molecule.
53. An isolated or recombinant mutant MarA family helix-turn-helix domain comprising a mutation in the helix-turn-helix domain most proximal to the amino terminus of the MarA family protein molecule.
54. A kit for identifying genetic loci in an microbe which affect resistance to compounds comprising a nucleotide sequence encoding a naturally occurring helix-turn-helix domain of a MarA family protein and mutant, inactive form of a MarA family protein helix-turn-helix domain.

The invention is further illustrated by the following examples, which should not be construed as further limiting.

### Examples

### Example 1. The identification of genetic loci in Mycobacrerium smegmatis which are involved in antibiotic resistance.

Multidrug resistance in Mycobacterium is presumed to occur via the accumulation of independent chromosomal mutations which affect susceptibility to individual drugs or a single plieotropic mutation, e.g., in *mar*. In *Escherichia coli* and other *Enterobacteriaceae* multidrug resistance is generally attributed to plasmids and transposons. Still, multidrug resistance can arise via derepression of the *E. coli may* (multiple antibiotic resistance) operon, either by mutation or exposure to inducing compounds (S.P. Cohen et al, J. Bacteriol., 1993, 175:1484-1492). In Mycobacterium, the observed relatively high frequency of multidrug resistance and the suggested relationship of inadequate treatment to the emergence of resistance (B.R. Bloom et al, Science, 1992, 257:10544-10642) fit with the selection of *E. coli* Mar mutants. The possible existence of a *mar*-like regulatory drug resistance response in *Mycobacterium M. smegmatis* mc²155 bearing pMV261:*marA* showed increased resistance to multiple antimicrobial agents, including rifampin, isoniazid, ethambutol, chloramphenicol, and tetracycline, compared to the microbe with vector alone (Table 3) when grown at 37°C but not at 30°C. Increased resistance to rifampin, however, was also noted at 30°C. Rifampin resistance also increased in the presence of vector alone at both temperatures, although this finding was variable. When it did occur, this was the only drug to which the vector appeared to affect parental susceptibility levels. Resistance of M. *smegmatis* to chloramphenicol increased two-fold and resistance to tetracycline increased nearly five-fold in the presence of *marA*. Ethambutol and isoniazid resistance increased 1.5-and 2.5-fold at 37°C. Little if any change in susceptibility to nalidixic acid, phenazine methosulfate, or sparfloxacin occurred; some increased susceptibility was observed for norfloxacin and streptomycin.

These changes in drug susceptibility were not seen with the *marA* gene cloned in the reverse orientation relative to the mycobacterial hsp60 promoter. Also, introduction of *marR* cloned with the same vector by PCR methods (primer nucleotide positions 1446 to 1462 and 1864 to 1879) caused no changes in susceptibility of *M. smegmatis* to any of the compounds tested (Table 3). Strains selected for spontaneous loss of plasmids by growth in the absence of kanamycin showed a return of the wild-type susceptibility phenotype. While multidrug resistance was clearly temperature dependent, and correlated with the presence of *marA* behind the heat shock promoter, it could reflect a resistance mechanism(s) per se which functions better at 37°C than at 30°C regardless of MarA expression. Of note, however, no temperature-dependent differences in susceptibility of wild-type cells were observed with any of the agents tested (Table 3).

### Example 2. Demonstration that the resistance phenotype in M. smegmatis was a direct result of MarA activity in the cell as demonstrated by insertional mutants targeted to the predicted helix-turn-helix domain

To obtain support for the notion that the resistance phenotype was a direct result of MarA activity in the cell, insertional mutants targeted to the first helix-turn-helix domain (HTH1) (M.N. Alekshun, et al. Chemother, 41:2067-2075) of the MarA protein were constructed. Megaprimer PCR (O.H. Landt, Gene 96:125-128) was used to introduce an *NheI* restriction site into the centers of both the helix A (position 1989) and helix B (position 2016) regions of *marA*. A double-stranded synthetic oligonucleotide with compatible ends was ligated to the *Nhe*I sites to produce two distinct insertional mutants interrupting each of the two putative alpha-helical regions (Fig. 1). These mutated genes were cloned into pMV261, as described above, for susceptibility testing in *M. smegmatis* at 37°C. They were also expressed from the isopropyl-β-D-thiogalactopyranoside (IPTG)-regulated T7 promoter resident on plasmid pET13a for testing in *E. coli* BL21 (Studier et al. 1990. Methods Enzymol. 185:60). Insertional inactivation of MarA at either helix A or helix B of the first HTH abolished the multidrug resistance phenotype in both *E. coli* and *M. smegmatis* the (Table 3 and 4).

To conform MarA expression, Northern blotting (S.K. Goda, Nucleic Acids Res. 23:3357-3358) was performed with total cellular RNA isolated by the TRIzol method (Gibco BRL, Gaithersburg, Md) from mid-log-phase cells grown at 30 and 37°C in Middlebrook 7H9-OADC medium following 1 h of pretreatment with lysozyme (4 mg of Tris-EDTA, pH 8.0, per ml) at 30°C. Equal amounts of RNA separated electrophoretically in 20 mM guanidine isothiocyanate, were probed with a radiolabeled marA PCR product. Hybridization signals were visualized with a PhosphorImager (Molecular Dynamics, Sunnyvale, Calif.).

MarA expression was observed in cells carrying the wild-type *E. coli marA* gene but not in the host carrying vector. Northern analysis was performed with PCR-amplified *marA* probe (nucleotide primers 1910 to 1893 and 2265 to 2282). The intensity of the *marA* hybridization signal was approximately fivefold higher in cells grown at 37 than at 30°C. As expected, a hybridization signal was detected in vector carrying *marA* in the reverse orientation, since a double-stranded *marA* probe was used.

Anti-MarA antiserum was prepared with MarA purified from BL21(DE3)pLysS cells (F.W. Studier, et al., Methods Enzymol, 1990, 185:60-89) bearing *marA* (M.N. Alekshun, Antimicrob. Agents Chemother, 1997, 41:2067-2075) cloned under the control of the T7 RNA polymerase initiation signals of pET13a. After induction with IPTG for 30 minutes, rifampin was added to maximize MarA synthesis. MarA was purified by a combination of the procedures of Li and Demple (Z. Li, et al, 1994, Biol. Chem., 1994, 269:18371-18377) and Langley et al. (K.E. Langley, et al., 1987, Euro. J. Biochem., 163:313-321). Anti-MarA rabbit antiserum was generated with purified MarA by Biodesign International (Kennebunk. Maine).

For Western analysis, cell lysates were prepared from mid-log-phase *M. smegmatis* or *E. coli* cultures by sonication in buffer (10 mM Tris-HCI, pH 8.0; 3C7O sodium dodecyl sulfate) on ice. Prior to electrophoresis, samples were treated by boiling for 5 min in sample buffer (125 mM Tris-HCI, pH 6.8; 20% glycerol; 6 mM β-mercaptoethanol: 0.05% bromphenol blue), and equivalent amounts of total protein were resolved by electrophoresis in a sodium dodecyl sulfate-17.5% polyacrylamide gel electrophoresis gel. Each lane contains 15µg ofmycobacteral protein from supernatant fractions. Proteins from *E*. *coli* AG100 and AG102 were used as negative and positive controls. Proteins were transferred to Immobilon-P membranes (Amersham) and analyzed by using rabbit anti-MarA antiserum and chemiluminescent detection (with a kit from New England Biolabs, Beverly Mass.).

A protein band migrating to the same place as purified MarA and having the expected molecular mass (14.3 kDa) was detected in MarA-containing cells grown at both temperatures (Fig. 2B); however, considerably more MarA was produced in cells incubated at 37°C. Since small amounts of MarA were detected at 30°C (Fig. 2B), the variable resistance to rifampin and the increased susceptibility phenotypes at 30°C may have been produced by relatively low cytoplasmic levels of MarA protein. By the same Western analysis. MarA was easily detected in *E*. *coli* and *M. smegmatis* lysates containing the mutant *marA* genes.

The mechanism of MarA-mediated multidrug resistance in *Mycobacterium* is unknown. The lack of a resistance phenotype mediated by the two different expressed mutant MarA proteins suggests that the multidrug resistance observed resulted from direct transcriptional activation of cognate promoters by MarA in *M. smegmatis*. Alternatively, MarA may have acted indirectly through induction of, or interaction with, endogenous proteins that mediate the mycobacterial Mar phenotype. In both instances, the multidrug resistance phenotype would have resulted from a *mar*-like regulatory system operating on other genes in this microbe. The effect, as with MarA in *E. coli,* may be linked to activation of a yet-to-be-discovered multidrug efflux system. The presence of efflux-like proteins (J.L. Doran, et al., 1997, Clin. Diagn. Lab. Immunol., 4:23-32 and J.H. Lui, et al., J. Bacteriol., 1996, 178:3791-3795), along with the earlier report of the existence of mycobacterial porin proteins (S.D. Mukhopadhyay, et al., J. Bacteriol., 1997, 179:6205-6207 and J.V. Trias, et al., Science, 1992, 258:1479-1481), indicate that, like in *E. coli*, effector proteins for *mar*-like multidrug resistance are present in *Mycobacterium*. The recently completed *Mycobacterium tuberculosis* genome sequencing project (W.J. Philipp, et al., Proc. Natl. Acad. Sci., 1996, 93:3132-3137) identified at least two proteins similar to MarA. Determination of whether these elements represent an endogenous *mar*-like system in this species awaits further study.

In addition to defining a function for MarA in a heterologous genus, our results are the first direct evidence of structurally important regions of MarA. The helix-turn-helix region targeted in site-directed mutagenesis corresponds to regions in the homologous proteins AraC and XylS (M.T. Gallegos, et al., Microbiol. Mol. Biol., Rev., 1997, 61:393-410), which are involved in DNA binding and transcriptional activation (A. Brunelle, et al. J. Mol. Biol., 1989, 209:607-622 and M.T. Gallegos, et al., J. Bacteriol, 1996, 178:6427-6434), Although the insertional mutations reported here involve significant changes to the wild-type protein, they point to the predicted helix-turn-helix domain as critical for protein function.

### Example 3. Development of a reporter gene screening assay for identifying compounds that reduce the activity of a MarA family protein helix-turn-helix domain.

The MarA family helix-turn-helix domain described in the previous examples is expressed in *E. coli* along with an *inaAl::phoA* reporter construct, made as previously described for *inaAl:lacZ* (Martin et al. 1995. J. Bacteriol. 177:4176), or a *micf:lacZ* reporter gene construct. The cells containing the phoA reporter construct are plated on media supplemented with 5-bromo-4-chloro-3-indolyl phosphate and containing kanamycin, while the cells containing the lacZ marker are grown on 5-bromo-4-chloro-3-indolyl β-D-galactoside with kanamycin. Colonies which turn blue, indicating transcription of the reporter gene construct, are isolated and placed in suspension. These cells are divided into two populations for treatment with each compound to be assayed: one test population to be treated with test compound and a control population to remain untreated. The appropriate population of cells are contacted with each compound to be tested. The cells are plated onto the same selective medium as indicated above. Colonies which turn blue indicate that the compound has no effect on the ability of the MarA helix-turn-helix domain to activate transcription of the reporter gene construct, while colonies which remain white after treatment with a compound indicate that the compound reduces the activity of the MarA helix-turn-helix domain.

### Example 4. Development of a screening assay for identifying compounds that increase the antibiotic sensitivity of an organism bearing a MarA family protein helix-turn-helix domain.

*M. smegmatis* mc²155 bearing pMV261::*marA*, which was shown above to have increased resistance to the antimicrobial agents: rifampin, isoniazid, ethambutol, chloramphenicol, and tetracycline is used to test for compounds which decrease this resistance. These cells are divided into two populations for treatment with each compound to be assayed: one test population to be treated with test compound and a control population to remain untreated. The appropriate population of cells is contacted with each compound to be tested. The treated and untreated cells are plated onto plates containing medium. Antibiotic sensitivity discs for the antibiotics listed above are placed on the plated cells and the plates are incubated. Compounds which reduce the zone of antibiotic sensitivity, i.e., show a larger zone of growth inhibition around the antibiotic discs from that seen in the cells which are not treated with compound are selected for identification.

### Example 5. Analysis of MarA expressed transcripts using DNA "chip" (GeneChip®) technology.

To identify all MarA induced/regulated transcripts in *Escherichia coli in vivo*, DNA "chip" (GeneChip®) technology is employed. DNA computer chips containing the entire *E. coli* chromosome are available through Affymetrix (Santa Clara, CA). In brief, *E. coli* containing a *marA* expression vector is induced in order to overexpress MarA *in vivo*. This treatment results in the activation of MarA regulated promoters. Total cDNA is prepared from these cells and used to probe the DNA chips. As a control, cDNA prepared from *E. coli* containing the expression vector lacking *marA* is used. This approach is also used to identify all genes expressed following exposure to any compound that induces *mar* expression.

### Example 6. Negative antisense control of MarA regulated loci.

Negative regulation of MarA responsive transcripts is achieved using a method similar to a previously described protocol (White et al., Antimicrob. Agents Chemother. 41:2699). *E. coli* is transformed with a vector encoding antisense-oligonucleotides complementary to 5'portions of the *marA, rob, soxS*, or other MarA family member protein transcripts following expression of these antisense-oligonucleotides *in vivo*. These structures interfere with translation of the *marA, rob, soxS*, etc. transcripts are targeted and degraded by endogenous RNaseH, an enzyme that degrades RNA-DNA hybrids. A vector which encodes antisense-oligonucleotides targeted toward all of the MarA homologs in *E. coli* is designed. Transfection of this vector into *E. coli* diminishes or eliminates the host's MarA family member regulated adaptational response to many antibiotics, disinfectants, organic solvents, and/or other environmental stimuli.

### Example 7. Resistance Pattern of E. coli microbes with plasmids bearing different E. coli mar genes.

Antibiotic MICs were determined for *E. coli* strains comprising different *E. coli mar* genes having mutations in the first helix-turn-helix domain. The antibiotic MICs are shown in Table 5. The A10 strain has an (Ala Ser Arg4 Ala Ser) inseration at amino acid 31. The A12 strain has a substitution of Val to Ala at position 33. The B7 strain has an AlsSerAla5Ser insertion at amino acid position 40. The B9 strain has a Trp to Ala substitution at position 42 and a His to Ser substitution at position 43. The Lys14 strain has a Lys to Gln substitution at position 41. The Trp15 strain has a Trp to Ala substitution at position 42. The Phe21 strain has a Phe to Leu substitution at position 48. The Ser2Leu3 strain has a Ser to Ala substitution at position 29 and a Leu to Ala substitution at position 30. The Ser2Ala strain has a Ser to Ala substitution at position 29.

### Example 8. Mutations in the second alpha helix of the first helix-turn-helix domain of MarA.

To determine the roles of the MarA HTH domains in more detail, additional mutants in these regions were devised. These mutants were tested for their ability to promote a Mar phenotype in E. coli and for changes in their affinity for the *mar* promoter. The mutants were synthesized in amounts comparable to the wild type MarA as determined by Western blot analysis. These mutants were defective in their ability to promote drug resistance, and exhibited lowered DNA binding affinities. Changes within the first alpha helix of the first HTH domain were less detrimental to MarA function than mutations in the second alpha helix of the first HTH domain.

### Example 9. Design of oligomers to create mutations in the second helix-turn-helix domain of MarA.

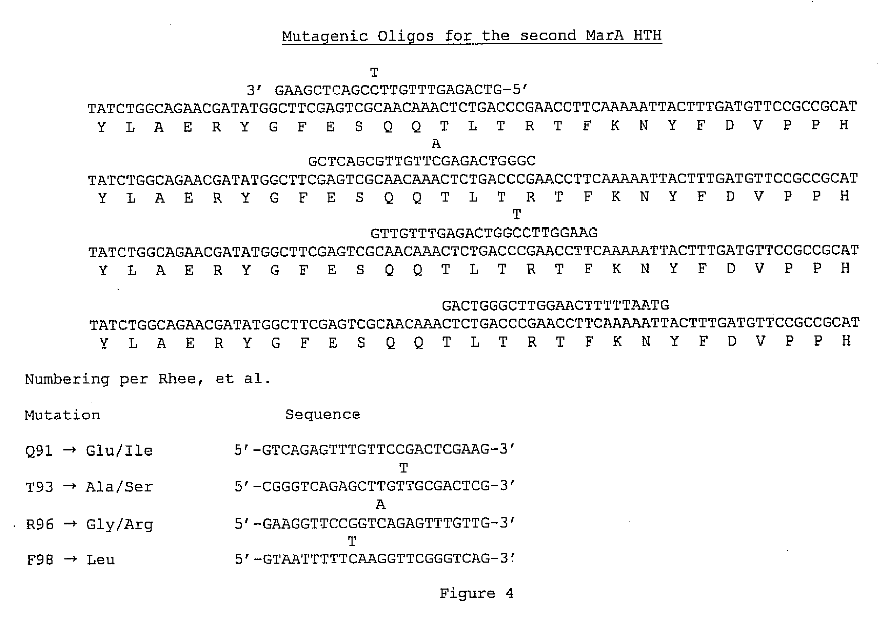

As was done for the first helix-turn-helix domain of MarA, mutagenic oligomers can be designed to make mutations in the second helix-turn-helix domain of MarA. Exemplary oligomers for mutating this region of MarA are illustrated in Figure 4.

### Equivalents

Those skilled in the art will recognize, or be able to ascertain using no more than routine experimentation, numerous equivalents to the specific polypeptides, nucleic acids, methods, assays and reagents described herein. Such equivalents are considered to be within the scope of this invention and are covered by the following claims.

**TABLE 1. Some Bacterial MarA homologs^{a}**

| Gram-negative | | Gram-positive |
|---|---|---|
| bacteria | | bacteria |
| *Escherichia coli* | | *Lactobacillus helveticus* |
| **MarA** (1) | *Kiebsiella pneumoniae* | **U34257** (39) |
| **OrfR** (2, 3) | **RamA** (27) | |
| **SoxS** (4, 5) | | *Azorhizobium caulinodans* |
| AfrR (6) | *Haemophilus influenzae* | S52856 (40) |
| AraC (7) | Ya52 (28) | |
| CelD (8) | | *Streptomyces spp.* |
| D90812 (9) | *Yersinia spp.* | **U21191** (41) |
| FapR (10, 11) | CafR (29) | AraL (42) |
| MelR (12) | LcrF (30) or VirF (30) | |
| ORF _*f*375 (13, 14 | | *Streptococcus mutans* |
| RhaR (15, 16, 17) | *Providencia stuartii* | MsmR (43) |
| | **AarP** (31) | |
| | | |
| RhaS (18) | | *Pediococcus pentosaceus* |
| Rob (19) | *Pseudomonas spp*. | RafR (44) |
| U73857 (20) | MmsR (32) | |
| XylR (21) | TmbS (33) | *Photobacterium leiognathi* |
| YijO (22) | XylS (34) | LumQ (45) |
| | Xys1,2,3,4 (35, 36) | |
| | | |
| *Proteus vulgaris* | | *Bacillus subtilis* |
| **PqrA** (23) | | AdaA (46) |
| | | YbbB (47) |
| *Salmonella typhimurium* | | YfiF (48) |
| | Cyanobacteria | YisR (49) |
| **MarA** (24) | *Synechocystis* spp. | YzbC (50) |
| InvF (25) | **LumQ** (38) | |
| PocR (26) | PchR (38) | |

| | | |
|---|---|---|
| ^{a} The smaller MarA homologs, ranging in size from 87 (U34257) to 138 (OrfR) amino acid residues, are represented in boldface. References are given in parentheses. | | |

### References for Table 1:

(1) S.P. Cohen, et al. 1993. J. Bacteriol. 175:1484-1492
(2) G.M. Braus, et al. 1984. J. Bacteriol. 160:504-509
(3) K. Schollmeier, et al., 1984. J. Bacteriol. 160:499-503
(4) C.F. Amabile-Cuevas, et al., 1991. Nucleic Acids Res. 19:4479-4484
(5) J. Wu, et al., 1991. J. Bacteriol. 173:2864-2871
(6) M.K. Wolf, et al., 1990. Infect. Immun. 58:1124-1128
(7) C.M. Stoner, et al. 1982. J. Mol. Biol. 153:649-652
(8) L.L. Parker, et al., 1990. Genetics 123:455-471
(9) H. Mori, 1996. Unpublished data taken from the NCBI databases
(10) P. Klaasen, et al., 1990. Mol. Microbiol. 4:1779-1783
(11) M. Ahmed, et al., 1994. J. Biol. Chjem 269-28506-28513
(12) C. Webster, et al., 1989. Gene 83:207-213
(13) G. Plunkett, , III. 1995. Unpublished
(14) C Garcia-Martin, et al., 1992. J. Gen. Microbiol. 138:1109-1116
(15) G. Plunkett, III., et al. 1993. Nucleic Acids Res. 21:3391-3398
(16) C. G. Tate, et al. 1992. J. Biol. Chem. 267:6923-6932
(17) J.F. Tobin et al., 1987. J. Mol. Biol. 196:789-799
(18) J. Nishitani, 1991. Gene 105:37-42
(19) R.E. Benz, et al., 1993. Zentralbl. Bakteriol. Parasitenkd. Infektionskr. Hyg. Abt.
(20) 1 Orig. 278:187-196
(21) M. Duncan, et al., 1996. Unpublished data
(22) H.J. Sofia, et al., 1994. Nucleic Acids Res. 22:2576-2586
(23) F.R. Blattner, et al., 1993. Nucleic Acids Res. 21:5408-5417
(24) H. Ishida, et al., 1995. Antimicrob. Agents Chemother. 39:453-457
(25) M.C. Sulavik, et al., 1997. J. Bacteriol. 179:1857-1866
(26) K. Kaniga, et al., 1994. Mol. Microbiol. 13:555-568
(27) J.R. Roth, et al. 1993. J. Bacteriol. 175:3303-3316
(28) A.M. George, et al., 1983. J. Bacteriol. 155:541-548
(29) R.D. Fleischmann, et al., 1995. Science 269:469-512
(30) E.E. Galyov, et al., 1991. FEBS Lett. 286:79-82
(31) N.P. Hoe, et al., 1992. J. Bacteriol. 174:4275-4286
(32) G. Comelis, et al., 1989. J. Bacteriol. 171:254-262
(33) D.R. Macinga, et al., 1995. J. Bacteriol. 177:3407-3413
(34) M.I. Steele, et al., 1992. J. Biol. Chem. 267:13585-13592
(35) G. Deho, et al., 1995. Unpublished data
(36) N. Mermod, et al., 1984. EMBO J. 3:2461-2466
(37) S.J. Assinder, et al., 1992. Nucleic Acids Res. 20:5476
(38) S.J. Assinder, et al., 1993. J. Gen. Microbiol. 139:557-568
(39) E.G. Dudley, et al., 1996. J. Bacteriol. 178:701-704
(40) D. Geelen, et al., 1995. Unpublished data
(41) J. Kormanec, et al., 1995. Gene 165:77-80
(42) C.W. Chen, et al., 1992. J. Bacteriol. 174:7762-7769
(43) R.R. Russell, et al., 1992. J. Biol. Chem, 267:4631-4637
(44) K.K. Leenhouts, et al., 1995. Unpublished data
(45) J.W. Lin, et al., 1995. Biochem. Biophys. Res. Commun. 217:684-695
(46) F. Morohoshi, et al. 1990. Nucleic Acids Res. 18:5473-5480
(47) M. Rosenberg, et al., 1979. Annu. Rev. Genet. 13:319-353
(48) H. Yamamoto, et al., 1996. Microbiology 142:1417-1421
(49) L.B. Bussey, et al., 1993. J. Bacteriol. 175:6348-6353
(50) P.G. Quirk, et al., 1994. Bichim. Biophys. Acta 1186:27-34

**Table 2. Bacterial strains and plasmids used in the Examples**

| Strain or plasmid | Description | Reference or Source |
|---|---|---|
| Strain | | |
| *E. coli* AG100 | Wild type | J. Bacteriol., 1983 155:531-540 |
| *E. coli* AG102 | Mar mutant of AG100 | J. Bacteriol., 1983 155:531-540 |
| *E. coli* BL21 | Expression strain for pET vectors | Novagen |
| *M. smegmatis* MC²155 | Electroporation competent | Mol. Microbiol, 1990, 4:1911-1919 |
| | | |

| Plasmids | | |
|---|---|---|
| pMV261 | *Mycobacterium-E*. *coli* shuttle vector | Nature, 1991, 351:456-460 |
| pPM10 | pMV261::*marA* | This study |
| pPM10R | pMV261::*marA* in antisense orientation | This study |
| pPM11 | pMV261::*marR* | This study |
| pPM1989R | pPM10 insertional mutant of helix A (Fig. 1) | This study |
| pPM2016A | pPM10 insertional mutant of helix B (Fig. 1) | This study |
| pET13a | T7 expression vector | Methods Enzymol, 1990, 185:60-89 |
| pEC10 | pET13::*marA* | This study |
| pEC1989R | pEC10 insertional mutuant of helix A | This s(Fig. 1) tudy |
| pEC2016A | pEC10 insertional mutuant of helix B | This study (Fig. 1) |

**Table 3. Antibiotic susceptibilities of M. smegmatis mc²155 microbes with and without plasmids bearing different E. coli mar genes**

| % Growth in antibiotic gradient^{a} | | | | | | | | | | | |
|---|---|---|---|---|---|---|---|---|---|---|---|
| | | RIF | | INH | | CML | | TET | | ETM | |
| *M. smegmatis microbe* | | 30°C | 37°C | 30°C | 37°C | 30°C | 37°C | 30°C | 37°C | 30°C | 37°C |
| Wild type | 11±1.2 | 11±1.1 | 21±1.2 | 20±1.0 | 20±0.8 | 22±0.9 | 20±1.9 | 19±12 | 40±2.2 | 44±2.1 | |
| Transformants bearing plasmids: | | | | | | | | | | | |
| pMV261 | 22±1.1 | 45±2.6 | 22±1.3 | 20±1.0 | 22±0.9 | 23±1.1 | 20±1.8 | 20±1.3 | 42±2.4 | 44±1.5 | |
| pPM10 | 68±1.6 | 90±2.1 | 15±1.5 | 45±2.2 | 13±1.1 | 46±1.6 | 15±2.1 | 95±3.1 | 38±1.8 | 63±2.7 | |
| pPM10R | 24±1.0 | 47±2.2 | 20±0.9 | 24±1.7 | 22±0.9 | 27±1.1 | 21±2.0 | 20±1.0 | 38+2.0 | 45±2.4 | |
| pPM11 | | 24±1.0 | 45±2.1 | 22±1.0 | 23±1.2 | 22±1.0 | 27±1.0 | 22±2.2 | 20±1.3 | 40±2.0 | 46±2.4 |
| pPM1989R | | ND | 10±1.0 | ND | 18±2.1 | ND | 26±1.4 | ND | 20±2.0 | ND | 48±2.2 |
| pPM2016A | | ND | 13±0.8 | ND | 22+1.0 | ND | 27±2.2 | ND | 22±1.7 | ND | 50±2.4 |

| | | | | | | | | | | | |
|---|---|---|---|---|---|---|---|---|---|---|---|
| ^{a}The antibiotics were tested in gradient plates at various concentrations (in micrograms per millilter) as follows: rifampin (RIF), 150; isoniazid (INH), 3.5; chloramphenicol (CML), 30; tetracycline (TET), 0.3; and ethambutol (ETM), 2.0 Values are means ± standard deviations of experiments performed in triplicate. ND, not determined. | | | | | | | | | | | |

**Table 4. Antibiotic susceptibilities of E. coli microbes with plasmids bearing different E. coli mar genes**

| % Gowth in antibiotic gradient^{a} | | | | |
|---|---|---|---|---|
| Plasmid borne by *E. coli* transformant | CML | TET | AMP | NAL |
| pET13a | 20±1.1 | 33±1.0 | 2±1.1 | 17±0.8 |
| pEC10 | 100 | 100 | 11±2.0 | 100 |
| pEC1989R | 20±1.6 | 33±1.4 | 2±1.0 | 17±1.2 |
| pEC2016A | 20±1.3 | 33±1.4 | 2±1.7 | 17±1.1 |

| | | | | |
|---|---|---|---|---|
| ^{a} The antibiotics were tested in gradient plates at various concentrations (in micrograms per milliliter) as follows: CML, 2.0; TET, 1.0:; ampicillin (AMP), 3.0; and nalidixic acid (NAL), 1.0 Values are means ± standard deviations of experiments performed in triplicate. | | | | |

**Table 5. Resistance Pattern ofMarA Mutants in 1st alpha Helix of First Helix-Turn-Helix Domain**

| Strain | Tet | Chlor | Nal Ac | Rif | Cipro | Nor | Amp | Genta | Ceph |
|---|---|---|---|---|---|---|---|---|---|
| A₁₀Ala₁₄ | 0.75±.25 | 1.0 | 0.38±.12 | 10.7±2.3 | <0.002 | <0.016 | 0.74±.02 | 1.0 | 1.5 |
| Ser₂Ala | 1.25±.43 | 3.3±.57 | 1.1±.38 | >32 | <0.002 | <0.016 | 2.4±.27 | 1.0 | 3.0 |
| pET13A | 0.71±.31 | 1.0 | 0.23±.03 | 13.3±4.6 | <0.002 | <0.016 | 0.76±.11 | 0.75 | 1.5 |
| A10 | 0.75±.25 | 1.08±.38 | 0.38±.13 | 12±4 | <0.002 | <0.016 | 0.8±.07 | 0.75 | 1.5 |
| A12 | 0.75±.25 | 1.5±0.5 | 0.54±.18 | 16 | <0.002 | <0.016 | 1.3±0.18 | 0.75 | 0.094 |
| B7 | 0.58±.14 | 1.3±.28 | 0.33±.14 | 17.3±6.1 | <0.002 | <0.016 | 0.9±.24 | 0.75 | 3 |
| B9 | 0.92±.52 | 1.2±.28 | 0.42±.06 | 15±2.3 | <0.002 | <0.016 | 0.84±.26 | 4 | 1.5 |
| Lys14 | 0.67±.14 | 1.2±.28 | 0.38±.12 | 13.3±2.3 | <0.002 | <0.016 | 0.9±.23 | 3 | 2 |
| Ser2Leu3 | 0.75 | 1.0 | 0.33±.07 | 14.6±2.3 | <0.002 | <0.016 | 0.75±.03 | 0.75 | 1.5 |
| Trp15 | 0.58±.14 | 0.83±.28 | 0.25±.12 | 10.6±2.3 | <0.002 | <0.016 | 0.84±.05 | 0.75 | 3 |
| A10Ala 6 | 0.92±.52 | 0.83±.14 | 0.37±.12 | 12±4 | <0.002 | <0.016 | 0.75±.03 | 0.75 | 1.5 |
| Phe21 | 0.66±.14 | 0.92±.14 | 0.33±.14 | 10.6±2.3 | <0.002 | <0.016 | 0.78±.04 | 1.0 | 2 |
| MarA | 1.2±.28 | 2.5±.86 | 0.96±56 | >32 | <0.002 | <0.016 | 3.0±.38 | 1.0 | 3 |
| BL21 | 0.75 | 0.66±.14 | 0.23±.03 | 6.7±1.1 | <0.002 | <0.016 | <0.125 | 1.0 | 1.5 |

| | | | | | | | | | |
|---|---|---|---|---|---|---|---|---|---|
| Data shown are the Avg ± SD (3 replicates). | | | | | | | | | |

## Claims

1. An antiinfective compound, wherein the antiinfective compound decreases an activity of a microbial transcription factor protein bearing a helix-turn-helix domain and reduces the infectivity or virulence of a microbe.

2. An antiinfective compound, wherein the antiinfective compound modulates an activity of a microbial transcription factor protein bearing a helix-turn-helix domain and reduces the infectivity or virulence of a microbe.

3. The antiinfective compound of claim 1 or 2, wherein the microbial transcription factor protein bearing a helix-turn-helix domain is a MarA family protein.

4. The antiinfective compound of claim 1 or 2, wherein the microbe is a Gram positive microbe.

5. The antiinfective compound of claim 1 or 2, wherein the microbe is of a genus selected from the group consisting of: *Enterobacteriaceae, Escherichia, Proteus, Klebsiella, Providencia, Enterobacter, Burkholderia, Pseudomonas, Aeromonas, Acinetobacter,* and *Mycobacteria*.

6. The antiinfective compound of claim 1 or 2, wherein the amino acid sequence of the helix-turn-helix domain comprises the sequence [KRQ]-[LIVMA]-X(2)-[GSTALIV]-{FYWPGDN}X(2)-[LIVMSA]-X(4,9)-[LIVMF]-X(2)-[LIVMSTA]-X(2)-[GSTACIL]-X(3)-[GANQRF]-[LIVMFY]-X(4,5)-[LFY]-X(3)-[FYIVA]-{FYWHCM}-X(3)-[GSADENQKR]-X-[NSTAPKL]-[PARL], where X is any amino acid.

7. The antiinfective compound of claim 3, wherein the MarA family protein is a protein selected from the group consisting of: XylS, AraC, MelR, RamA, AarP, Rob, SoxS and PqrA.

8. The antiinfective compound of claim 3, wherein the MarA family protein is MarA.

9. The antiinfective compound of claim 4, wherein the Gram positive microbe is of a genus selected from the group consisting of: *Enterococcus, Staphylococcus, Clostridium* and *Streptococcus*.

10. A pharmaceutical composition for reducing the infectivity or virulence of a microbe comprising a therapeutically effective amount of the compound of any one of the previous claims and a pharmaceutically acceptable carrier.

11. The pharmaceutical composition of claim 10, further comprising a second antimicrobial agent.

12. The pharmaceutical composition of claim 11, wherein the second antimicrobial agent is an antibiotic.

13. The pharmaceutical composition of claim 12, wherein the antibiotic is a fluoroquinolone.

14. A compound or pharmaceutical composition according to any one of claims 1 - 13 for use in the treatment of a microbial infection in a subject.

15. A compound or pharmaceutical composition according to any one of claims 1-13 for use in preventing a microbial infection in a subject.
